# EUROPÄISCHE PATENTSCHRIFT

(11) **EP 1 711 458 B1**
(45) Veröffentlichungstag und Bekanntmachung des Hinweises auf die Patenterteilung: **29.05.2013**
(21) Anmeldenummer: 05700969.8
(22) Anmeldetag: 17.01.2005
(51) Int. Cl.: C07C 277/08, C07C 279/04, C07D 233/48, C07D 233/28, C07C 211/15

(54) **VERFAHREN ZUR HERSTELLUNG VON GUANIDINIUM-SALZEN**
METHOD FOR PRODUCING GUANIDINIUM SALTS
PROCEDE DE PRODUCTION DE SELS DE GUANIDINIUM

(30) Priorität: 03.02.2004 DE 102004005404
(43) Veröffentlichungstag der Anmeldung: 18.10.2006
(73) Patentinhaber: Merck Patent GmbH, 64293 Darmstadt (DE)
(72) Erfinder: IGNATYEV, Nikolai, 47058 Duisburg (DE); WELZ-BIERMANN, Urs, 64646 Heppenheim (DE); BISSKY, German, 42119 Wuppertal (DE); WILLNER, Helge, 45481 Muelheim/Ruhr (DE)
(86) Internationale Anmeldenummer: PCT/EP2005/000389
(87) Internationale Veröffentlichungsnummer: WO 2005/075413

(56) Entgegenhaltungen:
- EP-A- 0 982 299
- WO-A-02/094822
- KANTLEHNER, WILLI ET AL: "Tetraalkylcyanoformamidinium salts and bis(dialkylamino)malonodinitriles from tetraalkylchloroformamidinium chlorides" SYNTHESIS , (5), 339-42 CODEN: SYNTBF; ISSN: 0039-7881, 1979, XP008044564
- CARPINO, LOUIS A. ET AL: "The Solid State and Solution Structure of HAPyU" JOURNAL OF ORGANIC CHEMISTRY , 66(15), 5245-5247 CODEN: JOCEAH; ISSN: 0022-3263, 2001, XP008044566
- ISOBE, TOSHIO ET AL: "2-Chloro-1,3-dimethylimidazolinium Chloride. 2. Its Application to the Construction of Heterocycles through Dehydration Reactions" JOURNAL OF ORGANIC CHEMISTRY , 64(19), 6989-6992 CODEN: JOCEAH; ISSN: 0022-3263, 1999, XP008044560
- DATABASE CA [Online] CHEMICAL ABSTRACTS SERVICE, COLUMBUS, OHIO, US; PRZYBYLSKI, JOZEF ET AL: "Preparation of 1,3-dimethyl-2-chloroethyleneuronium perchlorate as condensing agent for peptide bond formation" XP002322013 gefunden im STN Database accession no. 1997:280903 -& PL 170 332 B1 (UNIWERSYTET GDANSKI, POL.) 29. November 1996 (1996-11-29)
- KALINOWSKI, HANS OTTO ET AL: "Mesomeric cations, V. .pi.-Donor properties of oxygen and sulfur - dynamic NMR studies of aromatic uronium- and thiouronium salts and the question of planar inversion on oxygen and sulfur" CHEMISCHE BERICHTE , 112(4), 1153-67 CODEN: CHBEAM; ISSN: 0009-2940, 1979, XP008044568
- DATABASE CA [Online] CHEMICAL ABSTRACTS SERVICE, COLUMBUS, OHIO, US; MATSUO, HITOSHI: "Double layer capacitor" XP002322014 gefunden im STN Database accession no. 2002:693477 -& JP 2002 260966 A (ASAHI GLASS CO., LTD., JAPAN) 13. September 2002 (2002-09-13)
- MATEUS N M M ET AL: "SYNTHESIS AND PROPERTIES OF TETRA-ALKYL-DIMETHYLGUANIDINIUM SALTS AS A POTENTIAL NEW GENERATION OF IONIC LIQUIDS" GREEN CHEMISTRY, ROYAL SOCIETY OF CHEMISTRY, CAMBRIDGE,, GB, Bd. 5, 2003, Seiten 347-352, XP001166981 ISSN: 1463-9262 in der Anmeldung erwähnt
- SCHLAMA T ET AL: "ONE-STEP SYNTHESIS OF CHIRAL GUANIDINIUM SALTS FROM PHOSGENIMINIUM SALTS" JOURNAL OF ORGANIC CHEMISTRY, AMERICAN CHEMICAL SOCIETY. EASTON, US, Bd. 62, 1997, Seiten 4200-4202, XP002290887 ISSN: 0022-3263 in der Anmeldung erwähnt
- OTTO, MICHAEL ET AL: "Mono- and Diaminocarbenes from Chloroiminium and -amidinium Salts: Synthesis of Metal-Free Bis(dimethylamino)carbene" JOURNAL OF THE AMERICAN CHEMICAL SOCIETY , 126(4), 1016-1017 CODEN: JACSAT; ISSN: 0002-7863, 2004, XP008044561
- SACZEWSKI, FRANCISZEK ET AL: "2-Chloro-4,5-dihydroimidazoles. III. Synthesis, reactions and crystal structure of 2-(alkylthio)-7,8-dihydroimidazo[1,2-a]-1, 3,5-triazine-4(6H)- thiones", LIEBIGS ANNALEN DER CHEMIE ( 1987 ), (8), 721 -4 CODEN: LACHDL; ISSN: 0170-2041,

## Beschreibung

Die Erfindung betrifft ein zweistufiges Verfahren zur Herstellung von Guanidinium-Salzen mit einem Anion, ausgewählt aus der Gruppe Sulfonat, Alkyl- oder Aryl-Sulfat, Hydrogensulfat, Imid, Methanid, Carboxylat, Phosphat, Phosphinat, Phosphonat, Borat, Thiocyanat, Perchlorat, Fluorsilikat oder Nitrat und Zwischenverbindungen bei diesem Verfahren.

Guanidinium-Salze sind aufgrund ihrer Eigenschaften ideale Verbindungen zur Verwendung als ionische Flüssigkeiten, nicht-wässrige Elektrolyte, Phasentransferkatalysatoren oder grenzflächenaktive Stoffe. Das Gebiet der Ionischen Flüssigkeiten wird intensiv erforscht, da die Anwendungsmöglichkeiten vielfältig sind. Übersichtsartikel zu Ionischen Flüssigkeiten sind beispielsweise R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

Nach den klassischen Verfahren können Guanidinium-Salze durch Protonierung von Guanidinen mit starken Säuren oder durch Alkylierung von Guanidinen mit Alkylierungsreagenzien wie beispielsweise Alkyltriflat hergestellt werden. Die Guanidin-Einheit kann hierbei über vielfältige Methoden erhalten werden, beispielsweise durch Umsetzung von Aminen mit Thioharnstoffen, Chlorformamidiniumchloriden oder Aminoiminomethansulfonsäuren, wobei die Synthese von komplex substituierten Guanidinen oft kompliziert und/oder aufwendig ist (D. A. Powell, J. Org. Chem., 68 (2000), 2300-2309; D. H. R. Barton, J. Chem. Soc. Perkin Trans. I, (1982), 2085-2090).

Guanidinium-Chloride können auch direkt durch Reaktion von Phosgeniminiumchlorid mit einem sekundären Amin erhalten werden (T. Schlama et al, J. Org. Chem., 62 (1997), 4200-4202). Die Umsetzung zu Guanidinium-Salzen mit Anionen wie beispielsweise Hexafluorphosphat, Tetrafluorborat oder Bistrifluormethansulfonimidat entspricht einer Umsalzung, wie aus N.M.M Mateus et al, Green Chemistry, 5 (2003), 347-352 bekannt. Nachteilig bei dieser Umsalzung ist hierbei, dass die Endprodukte mit Chloridionen verunreinigt sind, da die Abtrennung der parallel entstehenden Ammoniumchloride oft sehr schwierig ist.

Die Aufgabe der vorliegenden Erfindung war daher, ein einfaches und kostengünstiges Verfahren zur Herstellung von Guanidinium-Salzen ohne Verwendung eines Guanidins als Edukt zur Verfügung zu stellen, welches Salze in hoher Reinheit liefert.

Diese Aufgabe wird durch das erfindungsgemäße Verfahren gelöst.

Überraschenderweise wurde gefunden, dass Guanidinium-Salze der Formel (1) 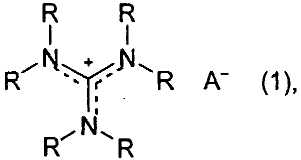 worin die Substituenten R jeweils unabhängig voneinander die Bedeutung von
Wasserstoff,
geradkettiges oder verzweigtes Alkyl mit 1-20 C-Atomen,
gesättigtes, teilweise oder vollständig ungesättigtes Cycloalkyl mit 3-7 C-Atomen, welches mit Alkylgruppen mit 1-6 C-Atomen substituiert sein kann, haben,
wobei ein oder mehrere Substituenten R teilweise oder vollständig durch Halogen oder teilweise durch CN oder NO₂ substituiert sein können und Halogen F, Cl, Br oder I bedeutet,
wobei bis zu vier Substituenten R paarweise durch Einfach- oder Doppelbindung miteinander verbunden sein können
und wobei ein Kohlenstoffatom oder zwei nicht benachbarte Kohlenstoffatome eines oder mehrerer Substituenten R durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
A⁻ ein Sulfonat, Alkyl- oder Aryl-Sulfat, Hydrogensulfat, Imid, Methanid, Carboxylat, Phosphat, Phosphinat, Phosphonat, Borat, Thiocyanat, Perchlorat, Fluorsilikat oder Nitrat ist,
mit der Maßgabe, dass nicht alle sechs Substituenten R gleichzeitig Wasserstoff sind,
durch Umsetzung einer Dihalogenverbindung der Formel (2) 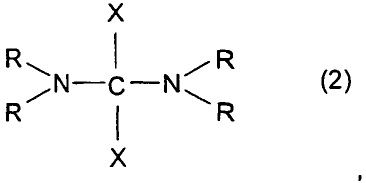 worin die Substituenten R eine bei Formel (1) angegebene Bedeutung haben und X F, Cl oder Br bedeutet,
mit der Maßgabe, dass nicht alle vier Substituenten R gleichzeitig Wasserstoff sind,
mit einer Verbindung der Formel (3)

Kt⁺ A⁻ (3),

worin A⁻ eine bei Formel (1) angegebene Bedeutung hat und Kt⁺ ein Proton, R"₃Si, Alkali- oder Erdalkalimetallkation, Ammoniumkation, Phosphoniumkation oder ein Kation der Gruppe 11 oder 12 sein kann, wobei R" jeweils unabhängig voneinander Phenyl oder eine lineare oder verzweigte Alkylgruppe mit 1-6 C-Atomen, die durch Phenyl substituiert sein kann, bedeutet,
und anschließender Reaktion der erhaltenen Verbindung der Formel (4) 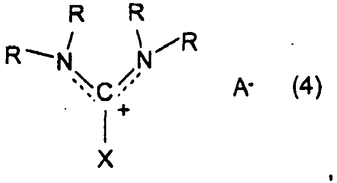 wobei die Substituenten R, X und A- eine bei Formel (1) oder (2) angegebene Bedeutung haben,
mit einer Verbindung der Formel (5) 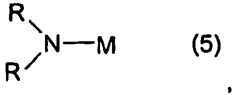 wobei die Substituenten R eine bei Formel (1) angegebene Bedeutung haben und
M Wasserstoff, ein Alkali- oder Erdalkalimetall bedeutet,
hergestellt werden können.

Louis A. Carpino et al, J. Org. Chem. 2001, 66, 5245-5247 beschreibt die Synthese von 1-(1-Pyrrolidinyl-1H-benzotriazol-1-ylmethylen)pyrroldinium Hexyfluorposphat durch Reaktion von Benzotriazol mit N,O-bis(trimethylsilyl)acetamid und anschließend mit Chlor-N,N,N',N'-bis(tetramehtylen)formamidinium hexafluorphosphat in Dichlormethan.

PL 170332 beschreibt die Verbindung 2-Chlor-1,3-dimethylimidazolidiniumperchlorat.

JP 2002260966 beschreibt generisch Verbindungen mit Imidazolium-, 3,4,5,6-Tetrahydro-pyrimidinium- und Imidazolidinium-Kationen in denen der Substituent R² zwischen den Stickstoffatomen der Heterocyclen F bedeuten kann und Anionen ausgewählt aus der Gruppe BF4⁻, PF6⁻, Cl⁻, CF₃SO₃⁻, AsF₆⁻, N(SO₂CF₃)₂⁻, NO₃⁻, ClO₄⁻, Br⁻ oder I⁻, sowie die Verbindungen 2-Fluor-1,3-dimethylimidazolium tetrafluorborat, 2-Fluor-3,4,5,6-tetrahydro-1,3-dimethylpyrimidinium tetrafluorborat und 2-Fluor-1,3-dimethylimidazolidinium tetrafluorborat.

EP 0982 299 A1 beschreibt Tetraalkylfluorformamidinium trifluoracetate.

Saczweski, Franciszek et al, Liebigs Annalen der Chemie, 1987, 8, 721-4 beschreibt die Verbindung 2-Chlor-4,5-dihydroimidazolium hydrogensulfat.

Als Substituenten R des Guanidinium-Kations kommen dabei neben Wasserstoff in Frage: C₁- bis C₂₀-Alkylgruppen, insbesondere Alkylgruppen mit 1, 2, 3, 4, 5, 6, 7, 8, 9, 10, 11 oder 12 C-Atomen, und gesättigte oder ungesättigte, d.h. auch aromatische, C₃- bis C₇-Cycloalkylgruppen, die mit C₁- bis C₆-Alkylgruppen substituiert sein können, insbesondere Phenyl. Die sechs Substituenten R des Guanidinium-Kations können dabei gleich oder verschieden sein, wobei nicht alle sechs Substituenten gleich Wasserstoff sein dürfen.

Die C₁-C₆-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl oder Hexyl. Gegebenenfalls Difluormethyl, Trifluormethyl, Pentafluorethyl, Heptafluorpropyl oder Nonafluorbutyl.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl, CN oder NO₂ substituiert sein kann.

Ferner können die Substituenten R ein oder zwei, einander nicht benachbarte Heteroatome oder Atomgruppierungen, ausgewählt aus der Gruppe O, C(O), C(O)O, S, S(O), SO₂, SO₂O, N, N=N, NH, NR', PR', P(O)R', P(O)R'O, OP(O)R'O und PR'₂=N enthalten, wobei R' ein nicht, teilweise oder perfluoriertes C₁- bis C₆-Alkyl, gesättigtes oder teilweise ungesättigtes C₃- bis C₇-Cycloalkyl, unsubstituiertes oder substituiertes Phenyl oder unsubstituierter oder substituierter Heterocyclus, sein kann.

Die Phenylgruppe kann hierbei durch C₁- bis C₆-Alkyl, C₁- bis C₆-Alkenyl, NO₂, F, Cl, Br, I, OH, C₁-C₆-Alkoxy, CN, SCN, SCF₃, SO₂CF₃, C(O)O-C₁-C₆-Alkyl, NH₂, C₁-C₆-Alkylamino oder C₁-C₆-Dialkylamino, COOH, C(O)NR'₂, SO₂OR', SO₂X', SO₂NR'₂, SO₃H oder NHC(O)R' substituiert sein, wobei X' F, Cl oder Br und R' eine zuvor angegebene Bedeutung hat, beispielsweise, o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-Aminophenyl, o-, m- oder p-(N,N-Dimethylamino)phenyl, o-, m- oder p-Nitrophenyl, o-, m- oder p-Hydroxyphenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

Als Heterocyclus wird ein gesättigter oder ungesättigter mono- oder bicyclischer heterocyclischer Rest mit 5 bis 13 Ringgliedem verstanden, wobei 1, 2 oder 3 N- und/oder 1 oder 2 S- oder O-Atome vorliegen können und der heterocyclische Rest ein- oder mehrfach durch C₁- bis C₆-Alkyl, C₁-bis C₆-Alkenyl, NO₂, F, Cl, Br, I, OH, C₁-C₆-Alkoxy, CN, SCN, SCF₃, SO₂CF₃, C(O)O-C₁-C₆-Alkyl, NH₂, C₁-C₆-Alkylamino oder C₁-C₆-Dialkylamino, COOH, C(O)NR'₂, SO₂OR', SO₂X', SO₂NR'₂, SO₃H oder NHC(O)R' substituiert sein kann, wobei X' und R' eine zuvor angegebene Bedeutung haben.

Der heterocyclische Rest ist vorzugsweise substituiertes oder unsubstituiertes 2- oder 3-Furyl, 2- oder 3-Thienyl, 1-, 2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3-oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1- oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinofinyl, 1-, 2-, 3-, 4-oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7- oder 8-Chinazolinyl oder 1-, 2- oder 3-Pyrrolidinyl.

Ohne Einschränkung der Allgemeinheit sind Beispiele für Substituenten R des Guanidinium-Kations:
- CH₃, -C₂H₅, -C₃H₇, -CH(CH₃)₂, -C₄H₉, -C(CH₃)₃, -C₅H₁₁, -C₆H₁₃, -C₇H₁₅, -C₈H₁₇, -C₉H₁₉, -C₁₀H₂₁, -C₁₂H₂₅, -C₂₀H₄₁, -OCH₃, -OCH(CH₃)₂, -CH₂OCH₃, -C₂H₄OCH(CH₃)₂, -SCH₃, -SCH(CH₃)₂, -C₂H₄SC₂H₅, -C₂H₄SCH(CH₃)₂, -S(O)CH₃, -SO₂CH₃, -SO₂C₂H₅, -SO₂C₃H₇, -SO₂CH(CH₃)₂, -CH₂SO₂CH₃, -CH₂N(H)C₂Hs, -C₂H₄N(H)C₂H₅, -CH₂N(CH₃)CH₃, -CN, -C₂H₄N(CH₃)CH₃, -N(CH₃)₂, -N(CH₃)C₃H₅, -N(CH₃)CF₃, -O-C₄H₈-O-C₄H₉, -S-C₂H₄-N(C₄H₉)2, -CF₃, -C₂F₅, -C₃F₇, -C₄F₉, -C(CF₃)₃, -CF₂SO₂CF₃, -C₂F₄N(C₂F₅)C₂F₅, -CHF₂, -CH₂CF₃, -C₂F₂H₃, -C₃FH₆, -CH₂C₃F₇, -C(CFH₂)₃, -CH₂C(O)OH, - CH₂C(O)CH₃, -CH₂C(O)C₂H₅, -CH₂C(O)OCH₃, CH₂C(O)OC₂H₅, -C(O)CH₃, - C(O)OCH₃,

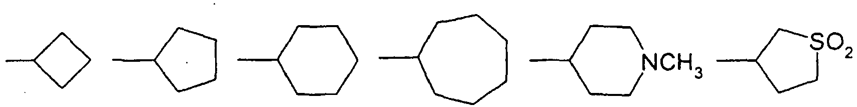

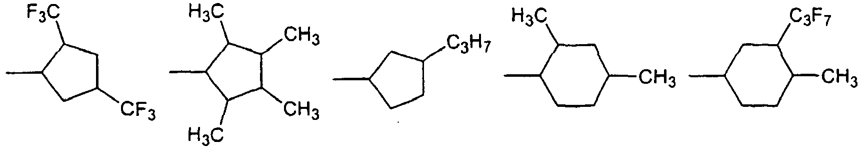

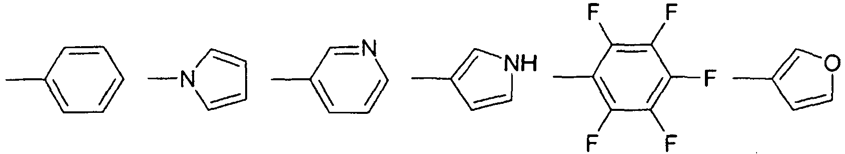

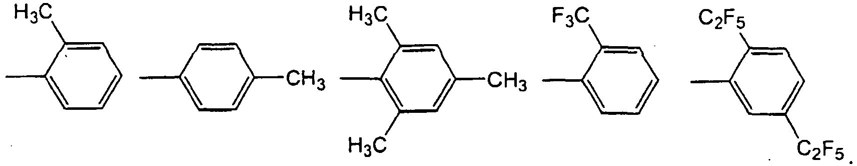

Bis zu vier Substituenten R können auch paarweise derart verbunden sein, dass mono-, bi- oder polycylische Kationen entstehen.

Ohne Einschränkung der Allgemeinheit sind Beispiele für solche Guanidinium-Kationen: 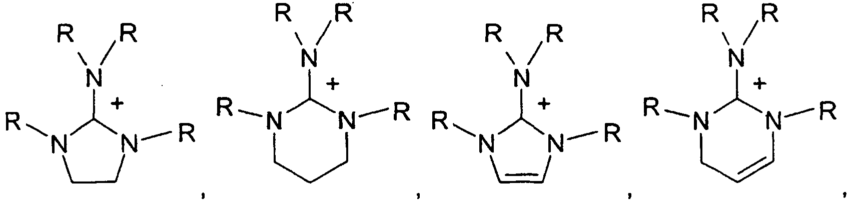 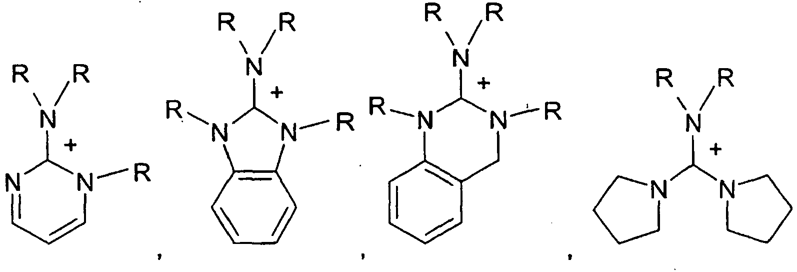 oder 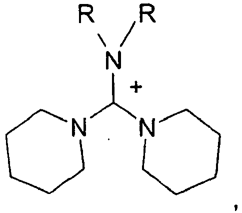 wobei die Substituenten R eine zuvor angegebene oder besonders bevorzugte Bedeutung haben können. Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, OH, C₁-C₆-Alkoxy, CN, SCN, SCF₃, SO₂CF₃, C(O)O-C₁-C₆-Alkyl, NH₂, C₁-C₆-Alkylamino oder C₁-C₆-Dialkylamino, COOH, C(O)NR'₂, SO₂OR', SO₂NR'₂, SO₂X', SO₃H oder NHC(O)R' substituiert sein, wobei X' und R' eine zuvor angegebene Bedeutung haben, substituiertes oder unsubstituiertes Phenyl oder unsubstituierter oder substituierter Heterocyclus substituiert sein.

Dihalogenverbindungen der Formel (2), 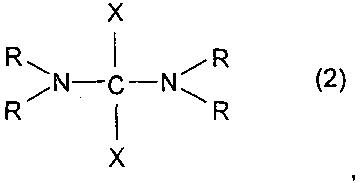 worin die Substituenten R eine bei Formel (1) angegebene oder eine bevorzugte Bedeutung haben und X F, Cl oder Br bedeutet,
mit der Maßgabe, dass nicht alle vier Substituenten R gleichzeitig Wasserstoff sind,
sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder aus K. Ohno et al., Heterocycles, 59 (2003), 317-322, A.A. Kolomeitsev et al., J. Fluorine Chem., 103 (2000), 159-162 oder H. Wittmann et al, Eur. J. Inorganic. Chem., 8 (2001), 1937-1948 bekannt sind. Dabei kann man auch von an sich bekannten, hier nicht näher erwähnten Varianten Gebrauch machen.

Besonders bevorzugt sind Verbindungen der Formel (2), wobei X F oder Cl bedeutet, ganz besonders bevorzugt sind Verbindungen der Formel (2), wobei X Cl bedeutet.

Die Verbindungen der Formel (3)

Kt⁺ A- (3),

worin A- eine bei Formel (1) angegebene Bedeutung hat und Kt⁺ ein Proton, R"₃Si, Alkali- oder Erdalkalimetallkation, Ammoniumkation, Phosphoniumkation oder ein Kation der Gruppe 11 oder 12 sein kann, wobei R" jeweils unabhängig voneinander Phenyl oder eine lineare oder verzweigte Alkylgruppe mit 1-6 C-Atomen, die durch Phenyl substituiert sein kann, bedeutet,
sind in der Regel ebenfalls kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart, Encyclopedia of Reagents for Organic Synthesis, Ed. Leo A. Paquette, John Wiley and Sons Ltd, 1995, EP 0929558 B1 oder US 6,423,454 für Fluoralkylphosphate, EP 1174941, EP 1205480 oder EP 1229038 für Fluoralkylborate bekannt sind. Dabei kann man auch von an sich bekannten, hier nicht näher erwähnten Varianten Gebrauch machen.

Kt⁺ ist beispielsweise NH₄⁺, H⁺, Li⁺, Na⁺, K⁺, Rb⁺, Mg²⁺, Ca²⁺, Ba²⁺, Cu⁺, Cu²⁺, Ag⁺, Zn²⁺, Cd²⁺, Hg⁺ oder Hg²⁺, besonders bevorzugt NH₄⁺, H⁺, Li⁺, Na⁺, K⁺, Rb⁺ oder Ca²⁺, wobei die Ladung in der jeweiligen Salzform der Formel (3) ausgeglichen vorliegt. Für ein zweiwertiges Kation werden zum Ladungsausgleich zwei einwertige Anionen der Formel A⁻ benötigt. Für den Ladungsausgleich eines zweiwertigen Anions werden zwei einwertige Kationen Kt⁺ benötigt. Die Maßgabe des Ladungsausgleichs gilt selbstverständlich auch für Verbindungen der Formel (1) und Formel (4).

Das Anion A- wird aus der Gruppe Sulfonat, Alkyl- oder Aryl-Sulfat, Hydrogensulfat, Imid, Methanid, Carboxylat, Phosphat, Phosphinat, Phosphonat, Borat, Thiocyanat, Perchlorat, Fluorsilikat oder Nitrat ausgewählt.

Bevorzugt werden Anionen A⁻ der Formeln
[R¹OSO₃]⁻, [R¹SO_{3]}⁻, [R^{F}SO_{3]}⁻, [(FSO₂)₂N]⁻, [(R^{F}SO₂)₂N]⁻, [(R^{F}SO₂)(R^{F}CO)N]⁻, [(R^{F}SO₂)₃C]⁻, [(FSO₂)₃C]⁻, [R¹CH₂C(O)O]⁻, [R^{F}C(O)O]⁻, [P(CₙF₂ₙ₊₁₋ₘHₘ)_{y}F_{6-y}]⁻, [P(C₆F₅)_{y}F_{6-y}]⁻, [(R¹O)₂P(O)O]⁻, [R¹₂P(O)O]⁻, [R¹P(O)O₂]²⁻, [R^{F}₂P(O)O]⁻, [R^{F}P(O)O₂]²⁻, [BF_{4-z}R^{F}_{z}]⁻, [BF_{4-z}(CN)_{z}]⁻, [B(C₆F₅)₄]⁻, [B(OR¹)₄]⁻, [N(CN)₂]⁻, [C(CN)₃]⁻, [N(CF₃)₂]⁻, [HSO₄]⁻, [SiF₆]²⁻, [ClO₄]⁻, [SCN]⁻ und [NO₃]⁻ ausgewählt,
worin die Substituenten R^{F} jeweils unabhängig voneinander die Bedeutung von
perfluoriertes und geradkettiges oder verzweigtes Alkyl mit 1-20 C-Atomen, perfluoriertes und geradkettiges oder verzweigtes Alkenyl mit 2-20 C-Atomen und einer oder mehreren Doppelbindungen,
perfluoriertes und gesättigtes, teilweise oder vollständig ungesättigtes Cycloalkyl mit 3-7 C-Atomen, das mit Perfluoralkylgruppen substituiert sein kann,
wobei die Substituenten R^{F} paarweise durch Einfach- oder Doppelbindung miteinander verbunden sein können und
wobei ein Kohlenstoffatom oder zwei nicht benachbarte Kohlenstoffatome des Substituenten R^{F}, die nicht α-ständig zu dem Heteroatom stehen, durch Atome und/oder Atomgruppierungen, ausgewählt aus der Gruppe - O-, -C(O)-, -S-, -S(O)-, -SO₂-, -N=, -N=N-, -NR'-, -PR'- und -P(O)R'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet,
worin die Substituenten R¹ jeweils unabhängig voneinander die Bedeutung von
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann, wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-, -P(O)R'-, P(O)R'O-, OP(O)R'O-, -PR'₂=N-, -C(O)NH-, -C(O)NR'-, -SO₂NH- oder -SO₂NR'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 0, 1, 2, 3, oder 4,
z 0, 1, 2, 3 oder 4 bedeuten.

Als organische Gruppen R^{F} bzw. R¹ des Anions kommen dabei in Frage: C₁- bis C₂₀-, insbesondere C₁- bis C₁₂-Alkylgruppen, C₂- bis C₂₀-, insbesondere C₂- bis C₁₂-Alkenylgruppen oder gesättigte oder ungesättigte, d.h. auch aromatische, C₃- bis C₇-Cycloalkylgruppen, die mit C₁- bis C₆-Alkylgruppen substituiert sein können, insbesondere Phenyl. Als organische Gruppen für R¹ kommen noch C₂- bis C₂₀-, insbesondere C₂-bis C₁₂-Alkinylgruppen in Frage.

Ein geradkettiges oder verzweigtes Alkenyl mit 2 bis 20 C-Atomen, wobei auch mehrere Doppelbindungen vorhanden sein können, ist beispielsweise Vinyl, Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Die Gruppen R^{F} sind perfluoriert, d.h. alle Kohlenstoffatome sind nicht mit Wasserstoff, sondern mit Fluoratomen abgesättigt. Die Gruppen R¹ können teilweise mit Halogenatomen, insbesondere mit F und/oder Cl, CN oder NO₂ substituiert sein.

Für den Fall, dass mehrere R^{F} bzw. R¹ in einem Anion vorhanden sind, können diese auch paarweise derart durch Einfach- oder Doppelbindungen verbunden sein, dass mono- oder bicyclische Anionen entstehen.

Ferner können die Substituenten R^{F} ein oder zwei, einander nicht benachbarte und nicht zum Heteroatom α-ständige Atome oder Atomgruppierungen, ausgewählt aus der Gruppe -O-, -C(O)-, -S-, -S(O)-, - SO₂-, -N=, -N=N-, -NR'-, -PR'- und -P(O)R'- enthalten, wobei R' ein nicht, teilweise oder perfluoriertes C₁- bis C₆-Alkyl, C₃- bis C₇-Cycloalkyl, ein unsubstituiertes oder substituiertes Phenyl, inklusive -C₆F₅, oder ein unsubstituierter oder substituierter Heterocyclus sein kann.

Ferner können die Substituenten R¹ ein oder zwei, einander nicht benachbarte und nicht zum Heteroatom α-ständige Atome oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-, -P(O)R'-, P(O)R'O-, OP(O)R'O-, -PR'₂=N-, -C(O)NH-, -C(O)NR'-, -SO₂NH- oder -SO₂NR'-enthalten, wobei R' ein nicht, teilweise oder perfluoriertes C₁- bis C₆-Alkyl, C₃- bis C₇-Cycloalkyl, ein unsubstituiertes oder substituiertes Phenyl, inklusive -C₆F₅, oder ein unsubstituierter oder substituierter Heterocyclus sein kann.

Ohne Einschränkung der Allgemeinheit sind Beispiele für Substituenten R¹ bzw. R^{F} des Anions:
- CH₃, -C₂H₅, -C₃H₇, -CH(CH₃)₂, -C₄H₉, -C(CH₃)₃, -C₅H₁₁, -C₆H₁₃, -C₇H₁₅, -C₈H₁₇, -C₉H₁₉, -C₁₀H₂₁, -C₁₂H₂₅, -C₂₀H₄₁, -CH₂OCH(CH₃)₂, -CH₂OCH₃, -C₂H₄OCH(CH₃)₂, -CH₂SCH₃, -CH₂SCH(CH₃)₂, -C₂H₄SC₂H₅, -C_{Z}H₄SCH(CH₃)₂, -CH₂S(O)CH₃, -CH₂SO₂CH₃, -C₂H₄SO₂C₂H₅, -C₂H₄SO₂C₃H₇, -CH₂SO₂CH(CH₃)₂, -CH₂SO₂CH₃, -CH₂OSO₂CH₃, -CH₂N(H)C₂H₅, -C₂H₄N(H)C₂H₅, -CH₂N(CH₃)CH₃, -C₂H₄N(CH₃)CH₃, -CH₂N(CH₃)₂, -C₂H₄N(CH₃)C₃H₅, -C₂H₄O-C₄H₈-O-C₄H₉, -C₂H₄S-C₂H₄-N(C₄H₉)₂, -CHF₂, -CH₂CF₃, -C₂F₂H₃, -C₃FH₆, -CH₂C₃F₇, -C(CFH₂)₃, -CH₂C(O)OH, -CH₂C(O)CH₃, -CH₂C(O)C₂H₅, -CH₂C(O)OCH₃, CH₂C(O)OC₂H₅, -C(O)CH₃, -C(O)OCH₃, -CH=CH₂, -C(CH₃)=CHCH₃, - CH₂CH=CHCH₃, -CH=CHN(CH₃)CH₃, 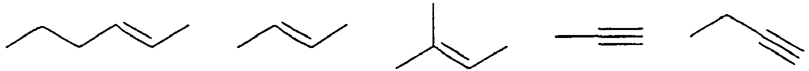 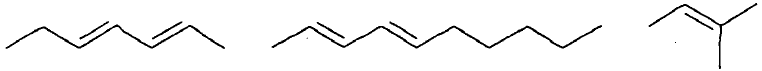 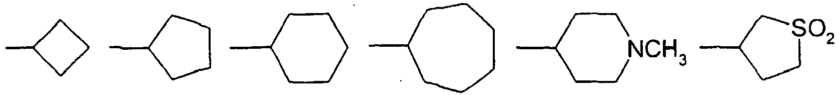 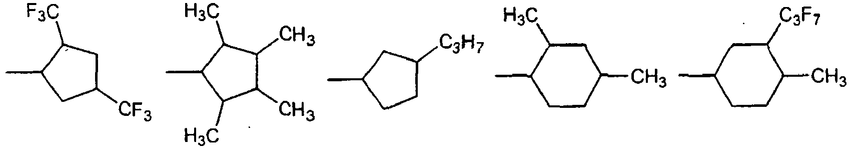 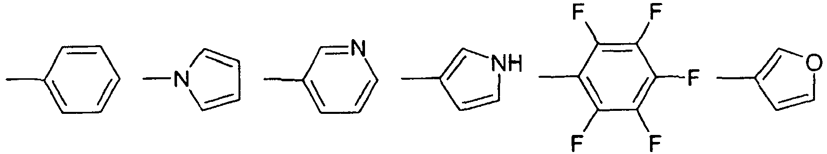 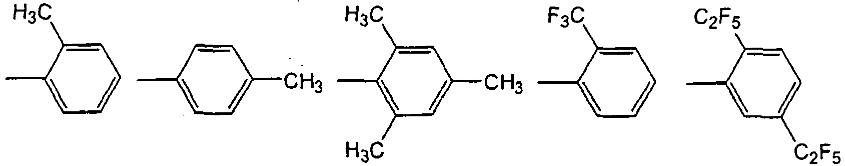 -CF₃, -C₂F₅, -C₃F₇, -C₄F₉, -C(CF₃)₃, -CF₂N(CF₃)CF₃, -CF₂OCF₃, - CF₂S(O)CF₃, -CF₂SO₂CF₃, -C₂F₄N(C₂F₅)C₂F₅, CF=CF₂, -C(CF₃)=CFCF₃, - CF₂CF=CFCF₃, -CF=CFN(CF₃)CF₃ oder -CF₂SO₂F.

Ohne Einschränkung der Allgemeinheit sind in folgenden einige Beispiele für Anionen A⁻ angegeben: [CH₃OSO₃]⁻, [C₂H₅OSO_{3]}⁻, [C(CN)₃]⁻, [CH₃SO₃]⁻, [C₈H₁₇SO₃]⁻, [CH₃C₆H₄SO₃]⁻, [CF₃SO₃]⁻, [C₂H₅SO₃]⁻, [CF₃CF₂SO₃]⁻, [(CF₃SO₂)₂N]⁻, [(FSO₂)₂N]⁻, [(CF₃SO₂)(CF₃CO)N]⁻, [(C₂F₅SO₂)(CF₃CO)N]⁻, [(C₂F₅SO₂)₂N]⁻, [(CF₃SO₂)₃C]⁻, [(C₂F₅SO₂)₃C]⁻, [(FSO₂)₃C]⁻, [CH₃C(O)O]⁻, [C₂H₅C(O)O]⁻, [CF₃C(O)O]⁻, [CF₃CF₂C₂O)O]⁻, [PF₆]⁻, [P(C₂F₅)₃F₃]⁻, [P(C₄F₉)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻, [P(C₃F₇)₃F₃]⁻, [P(C₂F₅)₂F₄]⁻, [(HO)₂P(O)O]⁻, [(CH₃O)₂P(O)O]⁻, [(C₂H₅O)₂P(O)O]⁻, [(C₂F₅)₂P(O)O]⁻, [(C₂F₅)P(O)O₂]²-, [P(C₆F₅)₂F₄]⁻, [(CH₃)₂P(O)O]⁻, [CH₃P(O)O₂]²⁻, [(CF₃)₂P(O)O]⁻, [CF₃P(O)O₂]²⁻, [BF₄]⁻, [BF₃(CF₃)]⁻, [BF₂(C₂F₅)₂]⁻, [BF₃(C₂F₅)]⁻, [BF₂(CF₃)₂]⁻, [B(C₂F₅)₄]⁻, [BF₃(CN)]⁻, [BF₂(CN)₂]⁻, [B(CN)₄]⁻, [B(OCH₃)₄]⁻, [B(CF₃)₄]⁻, [B(OCH₃)₂(OC₂H₅)₂]⁻, [B(O₂C₂H₄)₂]⁻, [B(O₂C₂H₂)₂]⁻, [B(O₂C₆H₄)₂]⁻, [N(CN)₂]⁻, [N(CF₃)₂]⁻, [HSO₄] ⁻, [ClO₄]⁻, [SiF₆]⁻, [SCN]⁻ oder [NO₃]⁻.

Bevorzugte Anionen A⁻ sind [CH₃OSO₃]⁻, [CH₃SO₃]⁻, [CF₃SO₃]⁻, [C₈H₁₇SO₃]⁻, [CH₃C₆H₄SO₃]⁻, [C₂F₅SO₃]⁻, [PF₆]⁻, [(C₂F₅)₃PF₃]⁻, [(C₂F₅)₂PF₄]⁻, [(C₄F₉)₃PF₃]⁻, [(C₃F₇)₃PF₃]⁻, [(HO)₂P(O)O]⁻, [(C₂F₅)₂P(O)O]⁻, [(CH₃O)₂P(O)O]⁻, [(C₂F₅)P(O)O₂]²⁻, [BF₄]⁻, [B(CN)₄]⁻, [B(CF₃)₄]⁻, [B(C₂F₅)F₃]⁻, [N(CN)₂]⁻, [N(CF₃)₂]⁻, [N(SO₂CF₃)₂]⁻, [HSO₄]⁻, [SiF₆]²⁻, [ClO₄]⁻, [SCN]⁻ oder [NO₃]⁻.

Bevorzugte Verbindungen der Formel (3) sind
Na[OSO₂OCH₃], Na[SO₃CH₃], HSO₃CH₃, Na[SO₃CF₃], HSO₃CF₃, Ca[SO₃CF₃]₂, (CH₃)₃Si[SO₃CF₃], CH₃C₆H₄SO₃H, Na[C₂H₅SO₃], C₂H₅SO₃H, Na[CF₃CF₂SO₃], C₂F₅SO₃H, Li[(CF₃SO₂)₂N], H[(CF₃SO₂)₂N], Li[(C₂F₅SO₂)₂N], Li[(CF₃SO₂)₃C], H[(CF₃SO₂)₃C], Li[(C₂F₅SO₂)₃C], K[(FSO₂)₃C], Na[CH₃C(O)O], CF₃COOH, Na[C₂H₅C(O)O], H[CF₃CF₂C(O)O], H[PF₆], H[P(C₂F₅)₃F₃], H[P(CF₃)₃F₃], H[P(C₂F₄H)(CF₃)₂F₃], H[P(C₂F₃H₂)₃F₃], H[P(C₂F₅)(CF₃)₂F₃], H[P(C₆F₅)₃F₃], H[P(C₃F₇)₃F₃], H[P(C₂F₅)₂F₄], H₃PO₄, Na[(CH₃O)₂P(O)O], H[(C₂F₅)₂P(O)O], Li₂[(C₂F₅)P(O)O₂], H₂[(C₂F₅)P(O)O₂], H[P(C₆F₅)₂F₄], Na[(CH₃)₂P(O)O], Na₂[CH₃P(O)O₂], H[(CF₃)₂P(O)O], H₂[CF₃P(O)O₂], Na[BF₄], NH₄[BF₄], K[BF₃(CF₃)], K[BF₂(C₂F₅)₂], K[BF₃(C₂F₅)], K[BF₂(CF₃)₂], K[B(C₂F₅)₄], K[BF₃(CN)], K[BF₂(CN)₂], Na[B(CN)₄], K[B(CN)₄], Li[B(OCH₃)₄], K[B(CF₃)₄], Li[B(OCH₃)₂(OC₂H₅)₂], Li[B(O₂C₂H₄)₂], Li[B(O₂C₂H₂)₂], Li[B(O₂C₆H₄)₂], Ag[C(CN)₃], Na[N(CN)₂], Rb[N(CF₃)₂], Na[SO₄CH₃], Na[HSO₄], [H₂SO₄], [H₂SiF₆], Li[ClO₄], Na[ClO₄], Na[SCN] oder H[NO₃].

Besonders bevorzugte Verbindungen der Formel (3) sind H[P(C₂F₅)₃F₃]*5 H₂O, Li[(CF₃SO₂)₂N], H[SO₃CF₃], Ca[(SO₃CF₃)₂], Na[OSO₂OCH₃], (CH₃)₃Si[SO₃CF₃], Na[ClO₄], H[SO₃C₆H₄CH₃], H[(CF₃SO₂)₂N], H₃PO₄, Na[(CH₃O)₂P(O)O], H[O(O)P(C₂F₅)₂], Rb[N(CF₃)₂], Na[SO₄CH₃] oder H₂SO₄.

Die Reaktion der Dihalogenverbindungen der Formel (2) mit Verbindungen der Formel (3) kann vorteilhaft in Wasser durchgeführt werden, wobei Temperaturen von 0°-150°C, bevorzugt 0°-40°C geeignet sind. Besonders bevorzugt wird die Reaktion bei Raumtemperatur durchgeführt.

Die Reaktion kann jedoch alternativ auch in organischen Lösungsmitteln bei Temperaturen zwischen -50° und 150°C stattfinden. Geeignete Lösungsmittel sind hier mit Wasser mischbare Lösungsmittel wie beispielsweise Dimethoxyethan, Acetonitril, Aceton, Tetrahydrofuran, Dimethylformamid, Dimethylsulfoxid, Dioxan, Propionitril, Methanol, Ethanol oder Isopropanol oder Gemische untereinander oder mit Wasser. Acetonitril ist ein bevorzugtes organisches Lösungsmittel.

Bevorzugt wird die Reaktion bei 0°-100°, besonders bevorzugt bei 10°-70°C, ganz besonders bevorzugt bei Raumtemperatur durchgeführt.

Die Reakiton der Dihalogenverbindungen der Formel (2) mit Verbindungen der Formel (3) kann auch ohne den Einsatz von Lösungsmitteln durchgeführt werden und zwar bei Temperaturen, bei der die Dihalogenverbindung der Formel (2) flüssig ist.

Die Reaktionsführung unter Schutzgasatmosphäre ist möglich und für oxidationsempfindliche Edukte zu bevorzugen.

Erfindungsgemäß werden die Verbindungen der Formel (2) mit Verbindungen der Formel (3) in äquimolaren Mengen oder mit einem Überschuß der Verbindung der Formel (3) umgesetzt. Bevorzugt wird ein Überschuß von 5 bis 20% an Verbindung der Formel (3) eingesetzt. Für die Reaktion der Verbindungen der Formel (3), die als Gegenion [N(CF₃)₂]⁻ haben, mit Dichlor- oder Dibromverbindungen der Formel (2), ist es vorteilhaft, die Verbindung der Formel (3) in mindestens zweimolarer Menge einzusetzen.

Die aus dieser ersten Reaktion hervorgehenden Verbindungen der Formel (4), 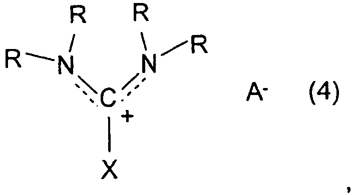 wobei die Substituenten R, X und A⁻ eine der zuvor angegebenen Bedeutungen oder bevorzugt angegebenen Bedeutungen haben, können mit sehr guter Ausbeute, in der Regel über 80%, vorzugsweise über 90%, isoliert werden. Vorteilhaft ist hierbei, dass die Abtrennung von den dabei entstehenden anorganischen Salzen KtX unproblematisch ist und die Verbindungen der Formel (4) nicht durch Halogenanionen X⁻ verunreinigt sind.

Die sich anschließende Umsetzung der Verbindungen der Formel (4) mit Ammoniak, Alkyl- oder Arylaminen oder -amiden der Formel (5) wird durch die hohe Elektrophilie des Carbokations angetrieben.

Die Verbindungen der Formel (5) 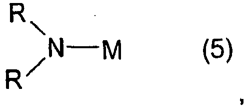 wobei die Substituenten R eine bei Formel (1) oder bevorzugt angegebene Bedeutung haben und
M Wasserstoff, ein Alkali- oder Erdalkalimetall bedeutet,
sind in der Regel ebenfalls kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder der Encyclopedia of Reagents for Organic Synthesis. Ed. Leo A. Paquette, John Wiley and Sons Ltd., 1995 bekannt sind. Dabei kann man auch von an sich bekannten, hier nicht näher erwähnten Varianten Gebrauch machen.

Die Substituenten R in Formel (5) sind jeweils unabhängig voneinander vorzugsweise Wasserstoff, geradkettiges oder verzweigtes Alkyl mit 1-20 C-Atomen oder gesättigtes, teilweise oder vollständig ungesättigtes Cycloalkyl mit 3-7 C-Atomen, welches mit Alkylgruppen mit 1-6 C-Atomen substituiert sein kann.

Bevorzugte Verbindungen der Formel (5) sind Ammoniak, Methylamin, Dimethylamin, Ethylamin, Diethylamin, Butylamin, Dibutylamin, Cyclohexylamin, Dicyclohexylamin oder Lithiumdimethylamid. Besonders bevorzugte Verbindungen der Formel (5) sind Ethylamin, Dimethylamin, Diethylamin, Dibutylamin, Dicyclohexylamin, oder Lithiumdimethylamid.

Die zweite Reaktion der Chlorformamidiniumsalze der Formel (4) mit Verbindungen der Formel (5) kann bei einer Temperatur durchgeführt werden, bei der mindestens eines der beiden Edukte flüssig ist. Auf den Einsatz von Lösungsmitteln kann dann vorteilhaft verzichtet werden. Bevorzugt findet die Reaktion bei Temperaturen von 15°-100°C, besonders bevorzugt bei 50°-70°C oder Raumtemperatur statt.

Die Reaktion kann jedoch alternativ auch in organischen Lösungsmitteln bei Temperaturen zwischen -50° und 150°C stattfinden. Geeignete Lösungsmittel sind hier mit Wasser mischbare Lösungsmittel wie beispielsweise Dimethoxyethan, Acetonitril, Aceton, Tetrahydrofuran, Dimethylformamid, Dimethylsulfoxid, Dioxan, Propionitril, Methanol, Ethanol oder Isopropanol oder Gemische untereinander oder mit Wasser. Bei Umsetzungen mit einem Silylamin wird bevorzugt ein nicht mit Wasser mischbares organisches Lösungsmittel verwendet, beispielsweise Dichlormethan oder Chloroform, bevorzugt Chloroform.
Bevorzugt wird die Reaktion bei 10°-70°C, besonders bevorzugt bei 40°-50°C oder Raumtemperatur, durchgeführt.
Die Reaktion kann jedoch ebenfalls vorteilhaft in Wasser durchgeführt werden, wobei Temperaturen von 0°-150°C geeignet sind. Reaktionen in Wasser finden bevorzugt bei Raumtemperatur statt.

Die Reaktionsführung unter Schutzgasatmosphäre ist möglich und für oxidationsempfindliche Edukte zu bevorzugen. Sie wird ebenfalls bei Normaldruck durchgeführt, wobei die Umsetzung beispielsweise mit Ammoniak, Methylamin oder Dimethylamin, das heißt gasförmigen oder leicht flüchtigen Verbindungen der Formel (5) vorteilhafterweise in geschlossenen Gefäßen erfolgt. Man kann Schritt 2 des erfindungsgemäßen Verfahrens auch unter Druck führen, wobei ein Druck bis 50 bar vorteilhaft sein kann.

Erfindungsgemäß werden die Verbindungen der Formel (4) mit Verbindungen der Formel (5) in äquimolarer Menge umgesetzt. Ein Überschuß an Verbindungen der Formel (5) kann vorteilhaft sein.

Die aus diesem zweiten Schritt hervorgehenden Guanidinium-Salze der Formel (1), wie zuvor beschrieben, können mit sehr guter Ausbeute, in der Regel über 80%, vorzugsweise über 90%, isoliert werden.

Nach dem erfindungsgemäßen Verfahren werden bevorzugt Guanidinium-Salze der allgemeinen Formel (1) 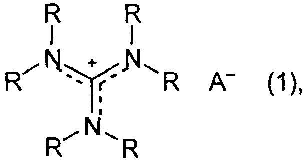 hergestellt, worin die Substituenten R jeweils unabhängig voneinander Wasserstoff oder eine geradkettige oder verzweigte Alkylgruppe mit 1-12 C-Atomen, insbesondere 1, 2, 3, 4, 5, 6, 7 oder 8 C-Atomen bedeuten, mit der Maßgabe, dass nicht alle sechs Substituenten R Wasserstoff sind,
oder mindestens zwei Substituenten R miteinander durch Einfach- oder Doppelbindungen miteinander verbunden sind, so dass ein monocyclisches Kation entsteht
und das Gegenanion A⁻ eine der bei Formel (3) oder eine bevorzugte oder ganz bevorzugte Bedeutung hat.

Nach dem erfindungsgemäßen Verfahren werden ganz besonders bevorzugt Guanidinium-Salze der Formel (1) hergestellt, wobei die Substituenten R jeweils unabhängig voneinander Methyl, Ethyl, n-Propyl, Isopropyl, n-Butyl, tert.-Butyl, sek.-Butyl, n-Hexyl, n-Octyl, Cyclohexyl oder Phenyl bedeuten oder zwei Substituenten R dermaßen miteinander verbunden sind, dass ein Imidazolidinium-Kation entsteht und das Gegenanion A⁻ eine der bei Formel (3) oder eine bevorzugte oder ganz bevorzugte Bedeutung hat.

Die nach der ersten Stufe des erfindungsgemäßen Verfahrens erhaltenen Zwischenverbindungen der Formel (4) sind ebenfalls aufgrund ihrer Eigenschaften geeignete Verbindungen zur Verwendung als ionische Flüssigkeiten.

Gegenstand der Erfindung sind daher ebenfalls die Zwischenverbindungen der Formel (4), 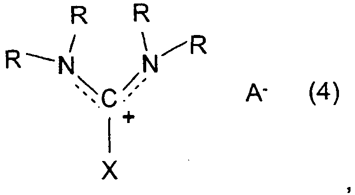 worin die Substituenten R jeweils unabhängig voneinander die Bedeutung von
Wasserstoff, geradkettiges oder verzweigtes Alkyl mit 1-20 C-Atomen, gesättigtes, teilweise oder vollständig ungesättigtes Cycloalkyl mit 3-7 C-Atomen, welches mit Alkylgruppen mit 1-6 C-Atomen substituiert sein kann, haben,
wobei ein oder mehrere Substituenten R teilweise oder vollständig durch Halogen oder teilweise durch CN oder NO₂ substituiert sein können und Halogen F, Cl, Br oder I bedeutet,
wobei bis zu vier Substituenten R paarweise durch Einfach- oder Doppelbindung miteinander verbunden sein können und wobei ein Kohlenstoffatom oder zwei nicht benachbarte Kohlenstoffatome eines oder mehrerer Substituenten R durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet,
X F, Cl oder Br bedeutet,
mit der Maßgabe, dass nicht alle vier Substituenten R gleichzeitig Wasserstoff sind und
A⁻ aus der Gruppe
[R¹OSO₃]⁻, [R¹SO₃]⁻, [R^{F}SO₃]⁻, [(FSO₂)₂N]⁻, [(R^{F}SO₂)(R^{F}CO)N]⁻, [(R^{F}SO₂)₃C]⁻, [(FSO₂)₃C]⁻, [R¹CH₂C(O)O]⁻, [P(CₙF₂ₙ₊₁₋ₘHₘ)_{y}F_{6-y}]⁻, [P(C₆F₅)_{y}F_{6-y}]⁻, [(R¹O)₂P(O)O]⁻, [R¹₂P(O)O]⁻, [R¹P(O)O₂]²⁻, [R^{F}₂P(O)O]⁻, [R^{F}P(O)O₂]²⁻, [BF_{4-z}R^{F}_{z}]⁻, [BF_{4-z}(CN)_{z}]⁻, [B(C₆F₅)₄]⁻, [B(OR¹)₄]⁻, [N(CN)₂]⁻, [C(CN₃)]⁻, [N(CF₃)₂]⁻, [SiF₆]²⁻ und [SCN]⁻ ausgewählt wird, wobei [CF₃SO₃]⁻ ausgenommen ist und
worin die Substituenten R^{F} jeweils unabhängig voneinander die Bedeutung von
perfluoriertes und geradkettiges oder verzweigtes Alkyl mit 1-20 C-Atomen, perfluoriertes und geradkettiges oder verzweigtes Alkenyl mit 2-20 C-Atomen und einer oder mehreren Doppelbindungen,
perfluoriertes und gesättigtes, teilweise oder vollständig ungesättigtes Cycloalkyl mit 3-7 C-Atomen, das mit Perfluoralkylgruppen substituiert sein kann,
wobei die Substituenten R^{F} paarweise durch Einfach- oder Doppelbindung miteinander verbunden sein können und
wobei ein Kohlenstoffatom oder zwei nicht benachbarte Kohlenstoffatome des Substituenten R^{F}, die nicht α-ständig zu dem Heteroatom stehen, durch Atome und/oder Atomgruppierungen, ausgewählt aus der Gruppe - O-, -C(O)-, -S-, -S(O)-, -SO₂-, -N=, -N=N-, -NR'-, -PR'- und -P(O)R'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet,
worin die Substituenten R¹ jeweils unabhängig voneinander die Bedeutung von
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann, wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-, -P(O)R'-, P(O)R'O-, OP(O)R'O-, -PR'₂=N-, -C(O)NH-, -C(O)NR'-, -SO₂NH- oder -SO₂NR'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und die Variablen
n 1 bis 20,
m 0, 1, 2 oder 3,
y 1, 2, 3 oder 4 und
z 1, 2, 3 oder 4 bedeuten.

Bevorzugt sind Verbindungen der Formel (4), worin die Substituenten R Wasserstoff oder eine geradkettige oder verzweigte Alkylgruppe mit 1-12 C-Atomen, insbesondere 1, 2, 3,4, 5, 6, 7 oder 8 C-Atomen bedeuten,
mit der Maßgabe, dass nicht alle vier Substituenten R Wasserstoff sind,
oder mindestens zwei Substituenten R miteinander durch Einfach- oder Doppelbindungen miteinander verbunden sind, so dass ein monocyclisches Kation entsteht und
das Gegenanion A⁻ [CH₃OSO₃]⁻, [C₂H₅OSO₃]⁻, [C(CN)₃]⁻,
[CH₃SO₃]⁻, [C₈H₁₇SO₃]⁻, [CH₃C₆H₄SO₃]⁻, [C₂H₅SO₃]⁻, [CF₃CF₂SO₃]⁻, [(FSO₂)₂N]⁻, [(CF₃SO₂)(CF₃CO)N]⁻, [(C₂F₅SO₂)(CF₃CO)N]⁻, [(CF₃SO₂)₃C]⁻, [(C₂F₅SO₂)₃C]⁻, [(FSO₂)₃C]⁻, [CH₃C(O)O]⁻, [C₂H₅C(O)O]⁻, [P(C₂F₅)₃F₃]⁻, [P(C₄F₉)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻, [P(C₃F₇)₃F₃]⁻, [P(C₂F₅)₂F₄]⁻, [(HO)₂P(O)O]⁻, [(CH₃O)₂P(O)O]⁻, [(C₂H₅O)₂P(O)O]⁻, [(C₂F₅)₂P(O)O]⁻, [(C₂F₅)P(O)O₂]²⁻, [P(C₆F₅)₂F₄]⁻, [(CH₃)₂P(O)O]⁻, [CH₃P(O)O₂]²⁻, [(CF₃)₂P(O)O]⁻, [CF₃P(O)O₂]²⁻, [BF₃(CF₃)]⁻, [BF₂(C₂F₅)₂]⁻, [BF₃(C₂F₅)]⁻, [BF₂(CF₃)₂]⁻, [B(C₂F₅)₄]⁻, [BF₃(CN)]⁻, [BF₂(CN)₂]⁻, [B(CN)₄]⁻, [B(OCH₃)₄]⁻, [B(CF₃)₄]⁻, [B(OCH₃)₂(OC₂H₅)₂]⁻, [B(O₂C₂H₄)₂]⁻_{,} [B(O₂C₂H₂)₂]⁻, [B(O₂C₆H₄)₂]⁻, [N(CN)₂]⁻, [N(CF₃)₂]⁻, [SiF₆]⁻ oder [SCN]⁻ bedeutet.

Ganz besonders sind Verbindungen der Formel (4) bevorzugt, wobei die Substituenten R jeweils unabhängig voneinander Methyl, Ethyl, n-Propyl, Isopropyl, n-Butyl, tert.-Butyl, sek.-Butyl, n-Hexyl, n-Octyl, Cyclohexyl oder Phenyl bedeuten oder zwei Substituenten R dermaßen miteinander verbunden sind, dass ein Imidazolidinium-Kation entsteht und das Gegenanion A⁻ [CH₃OSO₃]⁻, [CH₃SO₃]⁻, [C₈H₁₇SO₃]⁻, [CH₃C₆H₄SO₃]⁻, [C₂F₅SO₃]⁻, [(C₂F₅)₃PF₃]⁻, [(C₂F₅)₂PF₄]⁻, [(C₄F₉)₃PF₃]⁻, [(C₃F₇)₃PF₃]⁻, [(HO)₂P(O)O]⁻, (CH₃O)₂P(O)O]⁻, [(C₂F₅)₂P(O)O]⁻, [(C₂F₅)P(O)O₂]²⁻, [B(CN)₄] ⁻. [B(CF₃)₄]⁻, [B(C₂F₅)F₃]⁻, [N(CN)₂]⁻, [N(CF₃)₂]⁻, oder [SCN]⁻ bedeutet.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die NMR-Spektren wurden an Lösungen in deuterierten Lösungsmitteln bei 20°C an einem Bruker Avance 250 Spektrometer gemessen. Die Meßfrequenzen der verschiedenen Kerne sind: ¹H: 250,13 MHz, ¹⁹F: 235,357 MHz und ³¹P: 101.254 MHz. Die Methode der Referenzierung wird bei jedem Spektrum bzw. bei jedem Datensatz separat angegeben.

### Beispiel 1:

### A) 1,3-Dimethyl-2-chlorimidazolidinium Tris(pentafluorethyl)trifluorphosphat

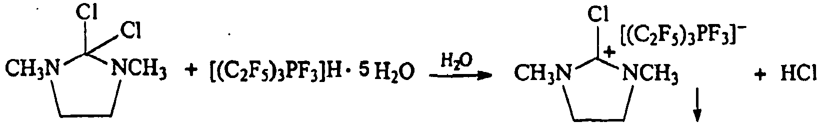

Zu einer Lösung von 80,0 g (0,473 mol) 2,2-Dichlor-1,3-dimethyl-imidazolidin in 300 ml Wasser werden unter Rühren bei Raumtemperatur 258,6 g (0,482 mol) Tris(pentafluorethyl)trifluorphosphorsäure Pentahydrat zugegeben. Es wird eine halbe Stunde gerührt und anschließend der Feststoff abfiltriert. Nach mehrmaligem Waschen mit 100 ml Wasser werden die entstandenen Kristalle im Vakuum von 10.0 Pa bei 60°C getrocknet. Man erhält 1,3-Dimethyl-2-chlorimidazolidinium Tris(pentafluorethyl)trifluorphosphat in einer Ausbeute von 95,9 % bezogen auf Dichlorimidazolidin.
Smp: 151-152°C
¹H NMR (Referenz: TMS intern; CD₃CN), ppm: 3.10s (2CH₃), 3.90 s (2CH₂).
¹⁹F NMR (Referenz: CCl₃F intern; CD₃CN), ppm: -43.57 d,m (PF), -79.62 m (CF₃), -81.31 m (2CF₃), -87.03 d,m (PF₂), -115.03 dm (CF₂) -115.62 dm (2CF₂); ¹J_{P,F} = 889 Hz, ¹J_{P,F} = 906 Hz, ²J_{P,F} = 87 Hz, ²J_{P,F} = 105 Hz.
³¹P NMR (Referenz: 85% H₃PO₄ extern; CD₃CN), ppm: -148.7 d,t,m.

### B) 1,3-Dimethyl-2-diethylaminoimidazolidinium

### Tris(pentafluorethyl)trifluorphosphat

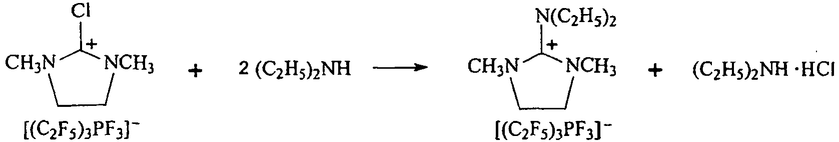

Innerhalb von 10 Minuten werden bei Raumtemperatur unter Rühren 63,5 g (0,868 mol) Diethylamin zu 167,5 g (0,289 mol) 1,3-Dimethyl-2-chlorimidazolidinium Tris(pentafluorethyl)trifluorphosphat gegeben. Die Reaktionsmischung wird 12 Stunden gerührt und anschließend das überschüssige Diethylamin abdestilliert. Der flüssige Rückstand wird mehrmals mit 100 ml Wasser gewaschen und anschließend unter Vakuum (10.0 Pa) bei 60°C getrocknet.
Man erhält 169,2 g 1,3-Dimethyl-2-diethylaminoimidazolidinium Tris(pentafluorethyl)trifluorphosphat, das entspricht einer Ausbeute von 95,2 %.
¹H NMR (Referenz: TMS intern; CD₃CN), ppm: 1.15 t (2CH₃), 2.90 s (2CH₃), 3.30 q (2CH₂), 3.63 s (2CH₂), ³J_{H,H} = 7.1 Hz.
¹⁹F NMR (Referenz: CCl₃F intern; CD₃CN), ppm: -43.59 d,m (PF), -79.65 m (CF₃), -81.35 m (2CF₃), -87.03 d,m (PF₂), -115.05 dm (CF₂) -115.63 dm (2CF₂); ¹J_{P,F} = 889 Hz, ¹J_{P,F} = 899 Hz, ²J_{P,F} = 87 Hz, ²J_{P,F} = 105 Hz.
³¹P NMR (Referenz: 85% H₃PO₄ extern; CD₃CN), ppm: -148.9 d,t,m.

### C) 1,3-Dimethyl-2-dibutylaminoimidazolidinium

### Tris(pentafluorethyl)trifluorphosphat

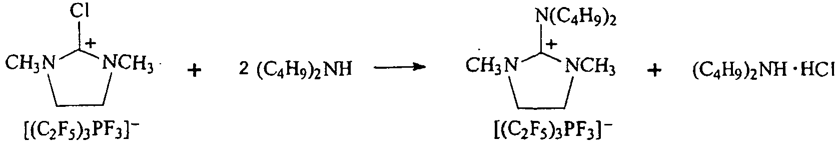

Innerhalb von 10 Minuten werden bei Raumtemperatur unter Rühren 13,4 g (103,7 mmol) Dibutylamin zu 20,0 g (34,6 mmol) 1,3-Dimethyl-2-chlorimidazolidinium Tris(pentafluorethyl)trifluorphosphat gegeben. Die Reaktionsmischung wird 12 Stunden bei Raumtemperatur und 2 Stunden bei 60°C gerührt. Der Überschuß an Dibutylamin wird durch Waschen mit Hexan entfernt. Der erhaltene Rückstand wird mehrmals mit 50 ml Wasser gewaschen und anschließend unter Vakuum (10.0 Pa) bei 60°C getrocknet.
Man erhält 22,1 g 1,3-Dimethyl-2-dibutylaminoimidazolidinium Tris(pentafluorethyl)trifluorphosphat, das entspricht einer Ausbeute von 95,1 %.
Smp.: 36-38°C
¹H NMR (Referenz: TMS intern; CD₃CN), ppm: 0.92 t (2CH₃), 1.30 m (2CH₂), 1.55 m (2CH₂), 2.91 s (2CH₃), 3.23 d,d (2CH₂), 3.64 s (2CH₂), ³J_{H,H} = 7.3 Hz.
¹⁹F NMR (Referenz: CCl₃F intern; CD₃CN), ppm: -43.64 d,m (PF), -79.68 m (CF₃), -81.37 m (2CF₃), -87.04 d,m (PF₂), -115.09 dm (CF₂) -115.62 dm (2CF₂); ¹J_{P,F} = 890 Hz, ¹J_{P,F} = 898 Hz, ²J_{P,F} = 89 Hz, ²J_{P,F} = 105 Hz.
³¹P NMR (Referenz: 85% H₃PO₄extern; CD₃CN), ppm: -149.0 d,t,m.

### D) 1,3-Dimethyl-2-aminoimidazolidinium

### Tris(pentafluorethyl)trifluorphosphat

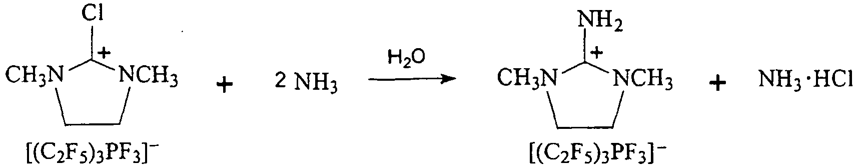

Zu einer Lösung von 2,0 g (3,46 mmol) 1,3-Dimethyl-2-chlorimidazolidinium Tris(pentafluorethyl)-trifluorphospat in 5 ml Wasser werden unter Rühren 1,2 g (17,62 mmol) einer 25%igen wässrigen Lösung von Ammoniak bei Raumtemperatur gegeben. Es wird 1 Stunde bei Raumtemperatur gerührt und abfiltriert. Der Niederschlag wird mehrmals mit 5 ml Wasser gewaschen und im Vakuum bei 7 Pa und 50-60°C getrocknet.
Man erhält 1,79 g 1,3-Dimethyl-2-aminoimidazolidinium Tris(pentafluorethyl)trifluorphosphat, das entspricht einer Ausbeute von 92,5 %.
Smp.: 67-68°C
¹H NMR (Referenz: TMS intern; CD₃CN), ppm: 2.87 s (2CH₃), 3.57 s (2CH₂), 6.25 br.s (NH₂).
¹⁹F NMR (Referenz: CCl₃F intern; CD₃CN), ppm: -43.57 d,m (PF), -79.61 m (CF₃), -81.30 m (2CF₃), -87.03 d,m (PF₂), -115.07 dm (CF₂) -115.62 dm (2CF₂); ¹J_{P,F} = 890 Hz, ¹J_{P,F} = 900 Hz, ²J_{P,F} = 86 Hz, ²J_{P,F} = 105 Hz.
³¹P NMR (Referenz: 85% H₃PO₄ extern; CD₃CN), ppm: -148.9 d,t,m.

### E) 1,3-Dimethyl-2-diethylaminoimidazolidinium

### Tris(pentafluorethyl)trifluorphosphat

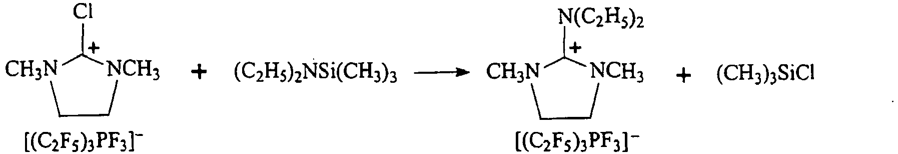

Innerhalb von wenigen Minuten werden bei Raumtemperatur unter Rühren 0,30 g (2,06 mmol) N,N-Diethyltrimethylsilylamin zu 1,0 g (1,73 mmol) 1,3-Dimethyl-2-chlorimidazolidinium Tris(pentafluorethyl)trifluorphosphat in 15 ml Chloroform gegeben. Die Reaktionsmischung wird 30 Minuten bei Raumtemperatur und 30 Minuten bei 40-50°C gerührt. Die flüchtigen Bestandteile werden abdestilliert und der erhaltene Rückstand unter Vakuum (7.0 Pa) bei 50°C getrocknet.
Man erhält 0,99 g 1,3-Dimethyl-2-diethylaminoimidazolidinium Tris(pentafluorethyl)trifluorphosphat, das entspricht einer Ausbeute von 93,0 %.
Smp.: 34-35°C
Die NMR-Spektren sind identisch zu denjenigen aus Beispiel 1B.

### Beispiel 2:

### A) Bis(dimethylamino)chlorcarbenium Tris(pentafluorethyl)trifluorphosphat

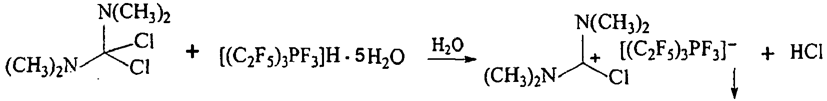

Zu einer Lösung von 20,0 g (0,117 mol) Bis(dimethylamino)dichlormethan in 100 ml Wasser werden unter Rühren bei Raumtemperatur 63,9 g (0,119 mol) Tris(pentafluorethyl)trifluorphosphorsäure Pentahydrat zugegeben. Es wird eine halbe Stunde gerührt und anschließend der Feststoff abfiltriert. Nach mehrmaligem Waschen mit 50 ml Wasser werden die entstandenen Kristalle im Vakuum von 10.0 Pa bei 60°C getrocknet. Man erhält 63,4 g Bis(dimethylamino)chlorcarbenium Tris(pentafluorethyl)trifluorphosphat, das entspricht einer Ausbeute von 93,3 %.
Smp.: 102-103°C
¹H NMR (Referenz: TMS intern; CD₃CN), ppm: 3.24 s (4CH₃).
¹⁹F NMR (Referenz: CCl₃F intern; CD₃CN), ppm: -43.57 d,m (PF), -79.62 m (CF₃), -81.31 m (2CF₃), -87.01 d,m (PF₂), -115.06 dm (CF₂) -115.60 dm (2CF₂); ¹J_{P,F} = 889 Hz, ¹J_{P,F} = 901 H_{z}, ²J_{P,F} = 85 Hz, ²J_{P,F} = 108 Hz.
³¹P NMR (Referenz: 85% H₃PO₄ extern; CD₃CN), ppm: -149.0 d,t,m.

### B) N,N,N',N'-Tetramethyl-N",N"-diethylguanidinium Tris(pentafluorethyl)-trifluorphosphat

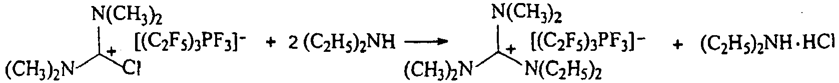

Innerhalb von 5 Minuten werden bei Raumtemperatur unter Rühren 4,53 g (61,9 mmol) Diethylamin zu 12,0 g (20,7 mmol)
Bis(dimethylamino)chlorcarbenium Tris(pentafluorethyl)trifluorphosphat gegeben. Die Reaktionsmischung wird 12 Stunden gerührt und anschließend das überschüssige Diethylamin abdestilliert. Der flüssige Rückstand wird mehrmals mit 30 ml Wasser gewaschen und anschließend unter Vakuum (10.0 Pa) bei 60°C getrocknet.
Man erhält 12,1 g N,N,N',N'-Tetramethyl-N",N"-diethylguanidinium Tris(pentafluorethyl)-trifluorphosphat, das entspricht einer Ausbeute von 94,7%.
Smp.: 28-30°C.
¹H NMR (Referenz: TMS intern; CD₃CN), ppm: 1.12 t (2CH₃), 2.85 s (2CH₃), 2.87 s (2CH₃), 3.20 m (2CH₂), ³J_{H,H} = 7.1 Hz.
¹⁹F NMR (Referenz: CCl₃F intern; CD₃CN), ppm: -43.57 d,m (PF), -79.63 m (CF₃), -81.32 m (2CF₃), -87.0 d,m (PF₂), -115.01 dm (CF₂) -115.62 dm (2CF₂); ¹J_{P,F} = 889 Hz, ¹J_{P,F} = 901 H_{Z}, ²J_{P,F} = 85 Hz, ²J_{P,F} = 105 Hz.
³¹P NMR (Referenz: 85% H₃O₄ extern; CD₃CN), ppm: -148.9 d,t,m.

### C)N,N,N',N'-Tetramethyl-N",N"-dibutylguanidinium Tris(pentafluorethyl)-trifluorphosphat

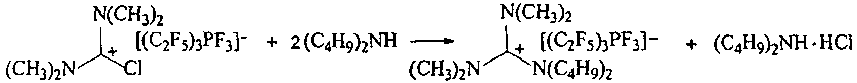

Innerhalb von 10 Minuten werden bei Raumtemperatur unter Rühren 13,4 g (103,7 mmol) Dibutylamin zu 20,0 g (34,4 mmol)
Bis(dimethylamino)chlorcarbenium Tris(pentafluorethyl)-trifluorphosphat gegeben. Die Reaktionsmischung wird 12 Stunden bei Raumtemperatur und 2 Stunden bei 60°C gerührt. Der Überschuß an Dibutylamin wird durch Waschen mit Hexan entfernt. Der flüssige Rückstand wird mehrmals mit 50 ml Wasser gewaschen und anschließend unter Vakuum (10.0 Pa) bei 60°C getrocknet.

Man erhält 22,6 g N,N,N',N'-Tetramethyl-N",N"-dibutylguanidinium Tris(pentafluorethyl)trifluorphosphat, das entspricht einer Ausbeute von 97,5 %.
¹H NMR (Referenz: TMS intern; CD₃CN), ppm: 0.90 t (2CH₃), 1.20-1.70 m (4CH₂), 2.87 s (2CH₃), 2.88 s (2CH₃), 3.12 m (2CH₂), ³J_{H,H} = 7.3 Hz.
¹⁹F NMR (Referenz: CCl₃F intern; CD₃CN), ppm: -43.62 d,m (PF), -79.67 m (CF₃), -81.36 m (2CF₃), -87.01 d,m (PF₂), -115.09 dm (CF₂) -115.62 dm (2CF₂); J_{P,F} = 889 Hz, ¹J_{P,F} = 898 Hz, ²J_{P,F} = 85 Hz, ²J_{P,F} = 105 Hz.
³¹P NMR (Referenz: 85% H₃PO₄ extern; CD₃CN), ppm: -148.9 d,t,m.

### D) N,N,N',N'-Tetramethyl-N",N"-dicyclohexylguanidinium Tris(pentafluorethyl)trifluorphosphat

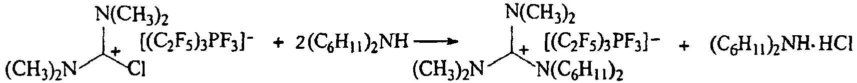

Innerhalb von 10 Minuten werden bei Raumtemperatur unter Rühren 18,7 g (103,1 mmol) Dicyclohexylamin zu 20,0 g (34,4 mmol) Bis(dimethylamino)chlorcarbenium Tris(pentafluorethyl)-trifluorphosphat gegeben. Die Reaktionsmischung wird 12 Stunden bei Raumtemperatur und 2 Stunden bei 60°C gerührt. Der Überschuß an Dicyclohexylamin wird durch Waschen mit Hexan entfernt. Der erhaltene Rückstand wird mehrmals mit 50 ml Wasser gewaschen und anschließend unter Vakuum (10.0 Pa) bei 60°C getrocknet.
Man erhält 22,8 g N,N,N',N'-Tetramethyl-N",N"-dicyclohexylguanidinium Tris(pentafluorethyl)trifluorphosphat, das entspricht einer Ausbeute von 91,3 %.
Smp.: 68-70°C
¹H NMR (Referenz: TMS intern; CD₃CN), ppm: 1.20-2.10 m (10 CH₂), 3.24 s (4CH₃), 6.26 t (2CH), J_{H,H} = 45.5 Hz.
¹⁹F NMR (Referenz: CCl₃F intern; CD₃CN), ppm: -43.64 d,m (PF), -79.70 m (CF₃), -81.40 m (2CF₃), -87.05 d,m (PF₂), -115.13 dm (CF₂) -115.69 dm (2CF₂); ¹J_{P,F} = 889 Hz, ¹J_{P,F} = 898 H, ²J_{P,F} = 87 Hz, ²J_{P,F} = 107 Hz.
³¹P NMR (Referenz: 85% H₃PO₄ extern; CD₃CN), ppm: -148.9 d,t,m.

### Beispiel 3:

### A) Bis(dimethylamino)chlorcarbenium Bis(trifluormethansulfonyl)imid

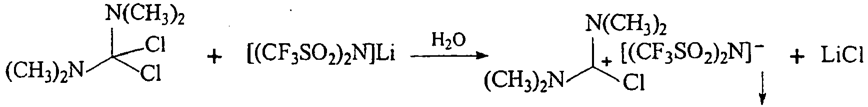

Zu einer Lösung von 80,0 g (0,468 mol) Bis(dimethylamino)dichlormethan in 300 ml Wasser werden unter Rühren bei Raumtemperatur 134,1 g (0,467 mol) Lithium Bis(trifluormethansulfonyl)imid in 200 ml Wasser zugegeben. Es wird eine halbe Stunde gerührt und anschließend der Feststoff abfiltriert. Nach mehrmaligem Waschen mit 100 ml Wasser werden die entstandenen Kristalle im Vakuum von 10.0 Pa bei 50°C getrocknet. Man erhält 165,5 g Bis(dimethylamino)chlorcarbenium Bis(trifluormethansulfonyl)imid, das entspricht einer Ausbeute von 85,1 %. Smp.: 68-70°C.
¹H NMR (Referenz: TMS intern; CD₃CN), ppm: 3.24 s (4CH₃).
¹⁹F NMR (Referenz: CCl₃F intern; CD₃CN), ppm: -78.87 s (2CF₃).

### B) Bis(dimethylamino)chlorcarbenium Bis(trifluormethansulfonyl)imid

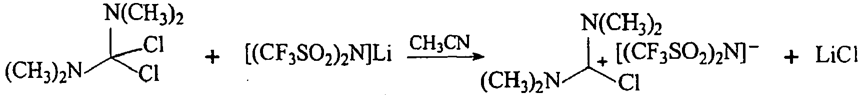

Zu einer Lösung von 0,5 g (2,92 mmol) Bis(dimethylamino)dichlormethan in 4 ml Acetonitril werden unter Rühren bei Raumtemperatur 0,84 g (2,92 mmol) Lithium Bis(trifluormethansulfonyl)imid in 4 ml Acetonitril zugegeben. Es wird 12 Stunden gerührt und anschließend der Feststoff LiCl abfiltriert. Acetonitril wird abdestilliert. Die entstandenen Kristalle werden im Vakuum von 10.0 Pa bei 50°C getrocknet. Man erhält 1,2 g Bis(dimethylamino)chlorcarbenium Bis(trifluormethansulfonyl)imid, das entspricht einer Ausbeute von 98,8 %.
Die Verbindung zeigt identische NMR-Spektren wie unter 3.A) hergestellt.

### C)N,N,N',N'-Tetramethyl-N",N"-dibutylguanidinium Bis(trifluormethansulfonyl)imid

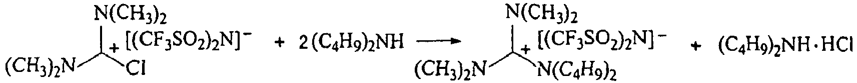

Innerhalb von 20 Minuten werden bei Raumtemperatur unter Rühren 141,3 g (1,1 mol) Dibutylamin zu 151,4 g (0,364 mol)
Bis(dimethylamino)chlorcarbenium Bis(trifluormethansulfonyl)imid gegeben. Die Reaktionsmischung wird 12 Stunden bei Raumtemperatur und 2 Stunden bei 60°C gerührt. Der Überschuß an Dibutylamin wird durch Waschen mit Hexan entfernt. Der flüssige Rückstand wird mehrmals mit 100 ml Wasser gewaschen und anschließend unter Vakuum (10.0 Pa) bei 60°C getrocknet.
Man erhält 183,1 g N,N,N',N'-Tetramethyl-N",N"-dibutylguanidinium Bis(trifluormethansulfonyl)imid, das entspricht einer Ausbeute von 98,9 %. ¹H NMR (Referenz: TMS intern; CD₃CN), ppm: 0.90 t (2CH₃), 1.20-1.70 m (4CH₂), 2.87 s (2CH₃), 2.88 s (2CH₃), 3.12 m (2CH₂), ³J_{H,H} = 7.3 Hz.
¹⁹F NMR (Referenz: CCl₃F intern; CD₃CN), ppm: -78.97 s (2CF₃).

### Beispiel 4:

### A) Bis(dimethylamino)chlorcarbenium Trifluormethansulfonat

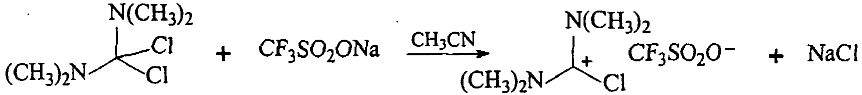

Zu einer Lösung von 2,0 g (11,7 mmol) Bis(dimethylamino)dichlormethan in 20 ml Acetonitril werden unter Rühren bei Raumtemperatur 2,01 g (11,7 mmol) Natriumtrifluormethansulfonat in 20 ml Acetonitril zugegeben. Es wird eine Stunde gerührt und 40 ml Diethylether zugegeben. Nach 12 Stunden Rühren wird anschließend der Feststoff NaCl abfiltriert. Das Lösungsmittel wird abdestilliert und die entstandenen Kristalle im Vakuum von 10.0 Pa getrocknet. Man erhält 3,29 g Bis(dimethylamino)chlorcarbenium Trifluormethansulfonat, das entspricht einer Ausbeute von 98,8 %.
Smp.: 93-96°C
¹H NMR (Referenz: TMS intern; CD₃CN), ppm: 3.24 s (4CH₃).
¹⁹F NMR (Referenz: CCl₃F intern; CD₃CN), ppm: -77.91 s (CF₃).

### B) Bis(dimethylamino)chlorcarbenium Trifluormethansulfonat

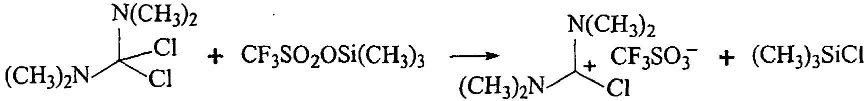

Zu 0,86 g (3,87 mmol) Trimethylsilyltrifluormethansulfonat, hergestellt durch Reaktion von 0,42 g Trimethylchlorsilan mit 0,58 g Trifluormethansulfonsäure bei Raumtemperatur, werden 0,5 g (2,92 mmol) Bis(dimethylamino)dichlormethan bei Raumtemperatur zugegeben. Die Reaktionsmischung wird 5 Minuten gerührt und anschließend alle flüchtigen Produkte im Vakuum entfernt. Der Rückstand wird im Vakuum bei 7 Pa und 50°C getrocknet. Man erhält 0,82 g Bis(dimethylamino)chlorcarbenium Trifluormethansulfonat, das entspricht einer Ausbeute von 98,7 %.
Smp.: 175-177°C
Die NMR-Spektren sind identisch zu denen von Beispiel 4.A).

### C) Hexamethylguanidinium Trifluormethansulfonat

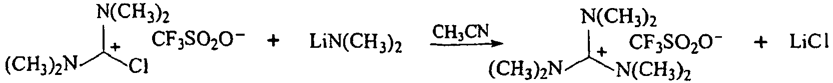

Zu 1,26 g (4,42 mmol) Bis(dimethylamino)chlorcarbenium trifluormethansulfonat in 20 ml Acetonitril werden bei Raumtemperatur 0,225 g (4,41 mmol) Lithiumdimethylamid zugegeben. Die Reaktionsmischung wird 12 Stunden gerührt und anschließend LiCl abfiltriert. Das Lösungsmittel wird abdestilliert und der Rückstand mit 20 ml Diethylether gewaschen und anschließend unter Vakuum (8.0 Pa) getrocknet.

Man erhält 1,25 g Hexamethylguanidinium Trifluormethansulfonat, das entspricht einer Ausbeute von 96,7 %.
¹H NMR (Referenz: TMS intern; CD₃CN), ppm: 2.89 s (6CH₃).
¹⁹F NMR (Referenz: CCl₃F intern; CD₃CN), ppm: -77.90 s (CF₃).

### Beispiel 5:

### A) 1,3-Dimethyl-2-chlorimidazolidinium Trifluormethansulfonat

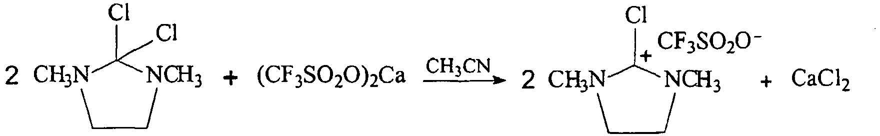

Zu einer Lösung von 5,0 g (29,6 mmol) 2,2-Dichlor-1,3-dimethylimidazolidin in 80 ml Acetonitril werden unter Rühren bei Raumtemperatur 5,0 g (14,8 mmol) Calciumtrifluormethansulfonat zugegeben. Es wird 12 Stunden gerührt und anschließend der Feststoff CaCl₂ abfiltriert. Acetonitril wird abdestilliert und der Rückstand mit 40 ml Diethylether gewaschen. Die entstandenen Kristalle werden im Vakuum von 10.0 Pa getrocknet. Man erhält 8,28 g 1,3-Dimethyl-2-chlorimidazolidinium Trifluormethansulfonat, das entspricht einer Ausbeute von 98,9 %.
Smp.: 62-63°C
¹H NMR (Referenz: TMS intern; CD₃CN), ppm: 3.10 s (2CH₃), 3.90 s (2CH₂).
¹⁹F NMR (Referenz: CCl₃F intern; CD₃CN), ppm: -77.88 s (CF₃).

### B) 1,3-Dimethyl-2-diethylaminoimidazolidinium Trifluormethansulfonat

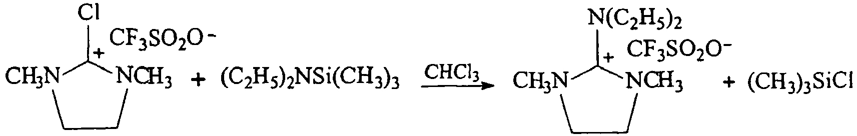

Innerhalb von wenigen Minuten werden bei Raumtemperatur unter Rühren 0,62 g (4,27 mmol) N,N-Diethyltrimethylsilylamin zu 1,0 g (3,54 mmol) 1,3-Dimethyl-2-chlorimidazolidinium Trifluormethansulfonat in 15 ml Chloroform gegeben. Die Reaktionsmischung wird 30 Minuten bei Raumtemperatur und 30 Minuten bei 40-50°C gerührt. Die flüchtigen Bestandteile werden abdestilliert und der erhaltene Rückstand unter Vakuum (7.0 Pa) bei 50°C getrocknet.
Man erhält 1,11 g 1,3-Dimethyl-2-diethylaminoimidazolidinium Trifluormethansulfonat als Öl, das entspricht einer Ausbeute von 98,2 %. ¹H NMR (Referenz: TMS intern; CD₃CN), ppm: 1.17 t (2CH₃), 2.92 s (2CH₃), 3.32 q (2CH₂), 3.65 s (2CH₂), ³J_{H,H} = 7.1 Hz.
¹⁹F NMR (Referenz: CCl₃F intern; CD₃CN), ppm: -78.02 s (CF₃).

### Beispiel 6: N,N,N',N'-Tetramethyl-N"-ethylguanidinium Tris(pentafluorethyl)trifluorphosphat

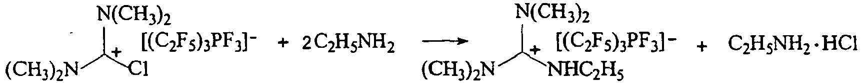

Zu 43,20 g (74,4 mmol) Bis(dimethylamino)chlorcarbenium Tris(pentafluorethyl)trifluorphosphat, hergestellt analog zu Beispiel 2.A), werden 24,0 g einer wässrigen Lösung (70%) von Ethylamin unter Rühren und Eiskühlung zugegeben. Die Reaktionsmischung wird anschließend 3 Stunden bei Raumtemperatur gerührt. Die Mischung wird mit 50 ml Wasser gewaschen und anschließend unter Vakuum (7.0 Pa) bei 60°C getrocknet. Man erhält 42,1 g der Flüssigkeit N,N,N',N'-Tetramethyl-N"-ethylguanidinium Tris(pentafluorethyl)-trifluorphosphat, das entspricht einer Ausbeute von 96,0 %.
¹H NMR (Referenz: TMS intern; CD₃CN), ppm: 1.21 t (CH₃), 2.21 s (4CH₃), 3.21 d,q (CH₂), 5.80 br.s (NH), ³J_{H,H} = 7.2 Hz, ³J_{H,H} = 5.6 Hz.
¹⁹F NMR (Referenz: CCl₃F intern; CD₃CN), ppm: -43.57 d,m (PF), -79.66 m (CF₃), -81.36 m (2CF₃), -86.97 d,m (PF₂), -115.05 dm (CF₂) -115.60 dm (2CF₂); ¹J_{P,F} = 889 Hz, ¹J_{P,F} = 901 Hz, ²J_{P,F} = 83 Hz, ² J_{P,F} = 105 Hz.
³¹P NMR (Referenz: 85% H₃PO₄ extern; CD₃CN), ppm: -148.9 d,t,m.

### Beispiel 7: 1,3-Dimethyl-2-ethylaminoimidazolidinium

### Tris(pentafluorethyl)trifluorphosphat

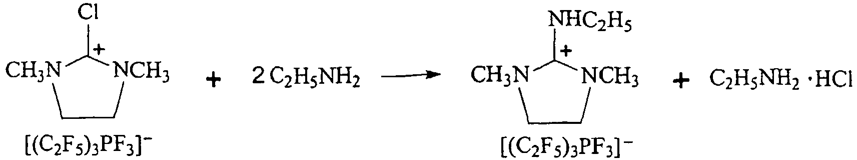

Zu 4,07 g (7,03 mmol) 1,3-Dimethyl-2-chlorimidazolidinium Tris(pentafluorethyl)trifluorphosphat, hergestellt analog zu Beispiel 1.A), werden 2,31 g einer wässrigen Lösung (70%) von Ethylamin unter Rühren und Eiskühlung zugegeben. Die Reaktionsmischung wird anschließend 3 Stunden bei Raumtemperatur gerührt. Die Mischung wird anschließend unter Vakuum (7.0 Pa) bei 60°C getrocknet.
Man erhält 3,76 g der Flüssigkeit 1,3-Dimethyl-2-ethylaminoimidazolidinium Tris(pentafluorethyl)-trifluorphosphat, das entspricht einer Ausbeute von 91,1 %.
¹H NMR (Referenz: TMS intern; CD₃CN), ppm: 1.25 t (CH₃), 2.96 s (2CH₃), 3.45 q (CH₂), 3.59 s (2CH₂), ³J_{H,H} = 7.3 Hz.
¹⁹F NMR (Referenz: CCl₃F intern; CD₃CN), ppm: -43.63 d,m (PF), -79.73 m (CF₃), -81.42 m (2CF₃), -87.07 d,m (PF₂), -115.07 dm (CF₂) -115.67 dm (2CF₂); ¹J_{P,F} = 890 Hz, ¹J_{P,F} = 901 Hz, ²J_{P,F} = 85 Hz, ² J_{P,F} = 105 Hz.
³¹P NMR (Referenz: 85% H₃PO₄ extern; CD₃CN), ppm: -148.9 d,t,m.

### Beispiel 8:

### A) Bis(dimethylamino)chlorcarbenium Perchlorat

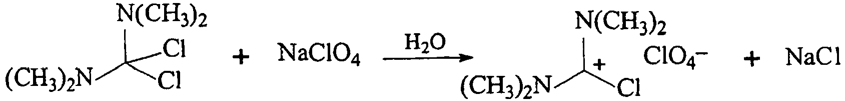

Zu einer Lösung von 5,00 g (29,2 mmol) Bis(dimethylamino)dichlormethan in 20 ml Wasser werden 3,60 g Natriumperchlorat in 10 ml Wasser unter Rühren zugegeben. Die Reaktionsmischung wird anschließend 1 Stunde unter Eiskühlung gerührt. Der Rückstand wird abfiltriert und mit 10 ml Eiswasser gewaschen und anschließend unter Vakuum (7.0 Pa) bei 50°C getrocknet.

Man erhält 6,64 g Bis(dimethylamino)chlorcarbenium Perchlorat, das entspricht einer Ausbeute von 94,0 %.
Smp.: 97-99 °C
¹H NMR (Referenz: TMS intern; CD₃CN), ppm: 3.27 s (4CH₃).

### B) N,N,N',N'-Tetramethyl-N",N"-diethylguanidinium Perchlorat

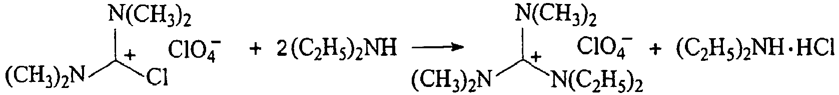

Zu 5,00 g (21,3 mmol) Bis(dimethylamino)chlorcarbenium Perchlorat werden 3,42 g (46,8 mmol) Diethylamin unter Rühren und Eiskühlung zugegeben. Die Reaktionsmischung wird anschließend 1 Stunde bei Raumtemperatur gerührt und anschließend 20 ml Wasser zugegeben. Die organische Phase wird abgetrennt und mit 20 ml Wasser gewaschen. Die Flüssigkeit wird anschließend unter Vakuum (7.0 Pa) bei 50°C getrocknet. Man erhält 5,17 g N,N,N',N'-Tetramethyl-N",N"-diethylguanidinium Perchlorat, das entspricht einer Ausbeute von 89,6 %.
¹H NMR (Referenz: TMS intern; CD₃CN), ppm: 1.15 t (2CH₃), 2.88 s (2CH₃), 2.91 s (2CH₃), 3.23 m (2CH₂), ³J_{H,H} = 7.2 Hz.

### Beispiel 9:

### A) Bis(dimethylamino)chlorcarbenium Tosylat

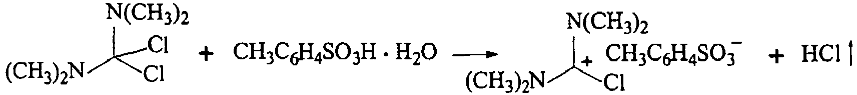

3,0 g (17,5 mmol) Bis(dimethylamino)dichlormethan und 3,33 g (17,5 mmol) p-Toluolsulfonsäure Monohydrat werden gemischt. Die Mischung wird innerhalb von 30 min auf 100°C erhitzt und anschließend ein Vakuum von 7 Pa über eine Stunde angelegt. Nach Abkühlung auf Raumtemperatur erhält man 5,25 g Bis(dimethylamino)chlorcarbenium Tosylat, das entspricht einer Ausbeute von 97,8 %.
Smp.: 122-126°C ¹H NMR (Referenz: TMS intern; CD₃CN), ppm: 2.33 s (CH₃), 3.24 s (4CH₃), 7.15, 7.18 (A,B; 2H), 7.58, 7.62 (A,B; 2H).

### B) Hexamethylguanidinium Tosylat

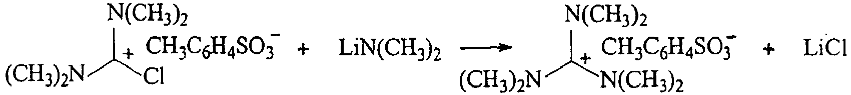

1,50 g (4,89 mmol) Bis(dimethylamino)chlorcarbenium Tosylat und 0,25 g (4,90 mmol) Lithiumdimethylamid werden in 15 ml Acetonitril unter Schutzgasatmosphäre (Argon) gelöst. Die Reaktionsmischung wird 5 Stunden bei Raumtemperatur gerührt und anschließend LiCl abfiltriert. Das Salz LiCl wird mit 5 ml Acetonitril gewaschen und die organischen Phasen vereinigt. Das Lösungsmittel wird abdestilliert und der Rückstand im Vakuum bei 7 Pa und 50°C getrocknet.
Man erhält 1,49g Hexamethylguanidinium Tosylat, das entspricht einer Ausbeute von 96,6 %.
Smp.: 103-104°C
¹H NMR (Referenz: TMS intern; CD₃CN), ppm: 2.33 s (CH₃), 2.86 s (6CH₃), 7.13, 7.16 (A,B; 2H), 7.57, 7.61 (A,B; 2H).

### Beispiel 10:

### A) Bis(dimethylamino)chlorcarbenium Hydrogensulfat

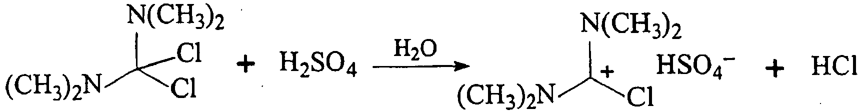

Zu einer Lösung von 5,65 g (33,0 mmol) Bis(dimethylamino)dichlormethan in 30 ml Wasser werden 3,24 g (33 mmol) Schwefelsäure unter Rühren und Eiskühlung zugegeben. Die Reaktionsmischung wird auf Raumtemperatur erwärmt. Alle flüchtigen Verbindungen werden im Vakuum (7.0 Pa) entfernt.
Man erhält 7,68 g hochviskoses Bis(dimethylamino)chlorcarbenium Hydrogensulfat, das entspricht einer nahezu quantitativen Ausbeute.
¹H NMR (Referenz: TMS intern; D₂O), ppm: 3.05 s (4CH₃).

### B) N,N,N';N'-Tetramethyl-N",N"-diethylguanidinium Hydrogensulfat

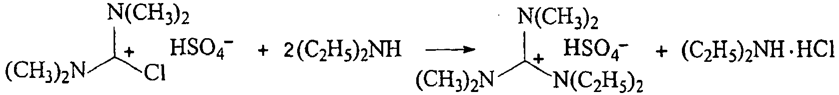

Zu 6,00 g (25,8 mmol) Bis(dimethylamino)chlorcarbenium Hydrogensulfat werden 4,20 g (57,4 mmol) Diethylamin unter Rühren und Eiskühlung zugegeben. Die Reaktionsmischung wird anschließend 1 Stunde bei Raumtemperatur gerührt und anschließend 50 ml Wasser zugegeben. Die organische Phase wird abgetrennt und die wässrige Phase mit 50 ml Dichlormethan extrahiert. Die vereinigten organischen Phasen werden mit 20 ml Wasser gewaschen und das Lösungsmittel im Vakuum abdestilliert. Der Rückstand wird anschließend unter Vakuum (7.0 Pa) bei 50°C getrocknet.
Man erhält 5,7 g viskoses N,N,N',N'-Tetramethyl-N",N"-diethylguanidinium Hydrogensulfat, das entspricht einer Ausbeute von 82,0 %.
¹H NMR (Referenz: TMS intern; D₂O), ppm: 0.99 t (2CH₃), 2.77 s (4CH₃), 3.13 m (2CH₂), ³J_{H,H} = 7.2 Hz.

### Beispiel 11:

### A) 1,3-Dimethyl-2-chlorimidazolidinium Nitrat

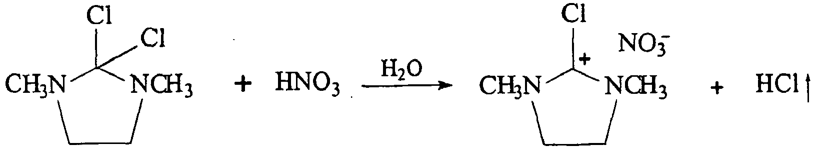

Zu 3,77 g (22,3 mmol) 2,2-Dichlor-1,3-dimethylimidazolidin werden unter intensivem Rühren 2,34 g (24,1 mmol) einer 65%igen Salpetersäure zugegeben. Diese Reaktionsmischung wird zunächst bei Raumtemperatur für eine Stunde bei 4 kPa und 20 min bei 7 Pa evakuiert. Nach Kühlung mit einem Eisbad wird nochmals 6 Stunden bei 7 Pa evakuiert. Man erhält 3,7 g 1,3-Dimethyl-2-chlorimidazolidinium Nitrat als Öl, das entspricht einer Ausbeute von 85,1%.
¹H NMR (Referenz: TMS intern; D₂O), ppm: 3.05 s (2CH₃), 3.89 s (2CH₂).

### B) 1,3-Dimethyl-2-dimethylaminoimidazolidinium Nitrat

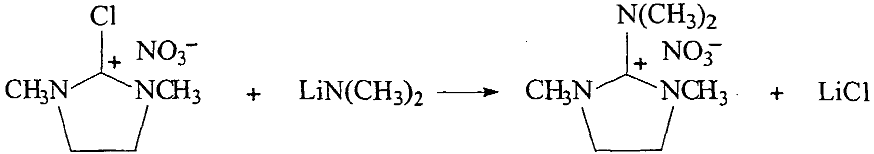

3,00 g (15,34 mmol) 1,3-Dimethyl-2-chlorimidazolidinium Nitrat und 0,78 g (15,29 mmol) Lithiumdimethylamid werden in 20 ml Acetonitril unter Schutzgasatmosphäre (Argon) gelöst. Die Reaktionsmischung wird 5 Stunden bei Raumtemperatur gerührt und anschließend LiCl abfiltriert. Das Salz LiCl wird mit 5 ml Acetonitril gewaschen und die organischen Phasen vereinigt. Das Lösungsmittel wird abdestilliert und der Rückstand im Vakuum bei 7 Pa und 50°C getrocknet.
Man erhält 2,91 g 1,3-Dimethyl-2-dimethylaminoimidazolidinium Nitrat als Flüssigkeit, das entspricht einer Ausbeute von 92,2 %.
¹H NMR (Referenz: TMS intern; CD₃CN), ppm: 2.96 s (2CH₃), 2.99 s (2CH₃), 3.63 s (2CH₂).

### Beispiel 12:

### A) Bis(dimethylamino)chlorcarbenium Trifluoracetat

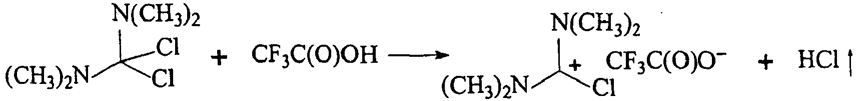

Zu 3,0 g (17,5 mmol) Bis(dimethylamino)dichlormethan werden unter Rühren 2,1 g (18,4 mmol) Trifluoressigsäure zugegeben. Die Reaktion wird eine Stunde bei Raumtemperatur gerührt und anschließend alle flüchtigen Produkte im Vakuum bei 7 Pa und 60°C entfernt. Man erhält 4,19 g Bis(dimethylamino)chlorcarbenium Trifluoracetat als sehr viskoses Öl, das entspricht einer Ausbeute von 96,1%.
¹H NMR (Referenz: TMS intern; CD₃CN), ppm: 3.27 s (4CH₃).
¹⁹F NMR (Referenz: CCl₃F intern; CD₃CN), ppm: -75.63 s (CF₃).

### B) Hexamethylguanidinium Trifluoracetat

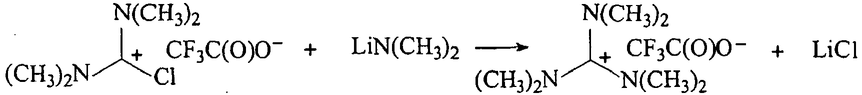

1,85 g (7,44 mmol) Bis(dimethylamino)chlorcarbenium Trifluoracetat und 0,38 g (7,45 mmol) Lithiumdimethylamid werden in 15 ml Acetonitril unter Schutzgasatmosphäre (Argon) gelöst. Die Reaktionsmischung wird 5 Stunden bei Raumtemperatur gerührt und anschließend LiCl abfiltriert. Das Salz LiCl wird mit 5 ml Acetonitril gewaschen und die organischen Phasen vereinigt. Das Lösungsmittel wird abdestilliert und der Rückstand im Vakuum bei 7 Pa und 50°C getrocknet.
Man erhält 1,81 g Hexamethylguanidinium Trifluoracetat als viskoses Öl, das entspricht einer Ausbeute von 99,2 %.
¹H NMR (Referenz: TMS intern; CD₃CN), ppm: 2.89 s (6CH₃).
¹⁹F NMR (Referenz: CCl₃F intern; CD₃CN), ppm: -75.56 s (CF₃).

### Beispiel 13:

### A) Bis(dimethylamino)chlorcarbenium Thiocyanat

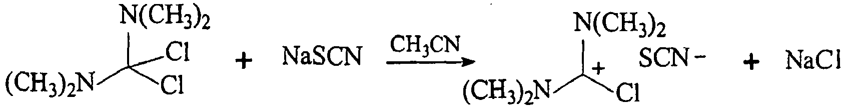

Zu einer Lösung von 3,0 g (17,5 mmol) Bis(dimethylamino)dichlormethan in 50 ml Acetonitril werden 2,84 g (35,0 mmol) Natriumthiocyanat bei Raumtemperatur zugegeben. Die Reaktionsmischung wird 24 Stunden gerührt und anschließend der Niederschlag NaCl abfiltriert. Anschließend wird das Lösungsmittel abdestilliert und der Rückstand mehrmals mit 50 ml Dichlormethan extrahiert. Nach Destillation des Dichlormethans wird der Rückstand bei 60°C und 7 Pa getrocknet. Man erhält 2,99 g der Flüssigkeit Bis(dimethylamino)chlorcarbenium Thiocyanat, das entspricht einer Ausbeute von 88,2%.
¹H NMR (Referenz: TMS intern; CD₃CN), ppm: 3.27 s (4CH₃).

### B) Hexamethylguanidinium Thiocyanat

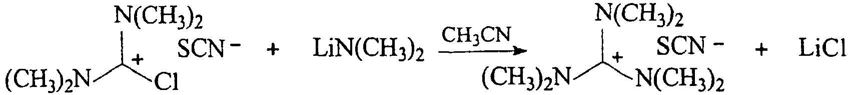

1,00 g (5,16 mmol) Bis(dimethylamino)chlorcarbenium Thiocyanat und 0,26 g (5,10 mmol) Lithiumdimethylamid werden in 10 ml Acetonitril unter Schutzgasatmosphäre (Argon) gelöst. Die Reaktionsmischung wird 5 Stunden bei Raumtemperatur gerührt und anschließend LiCl abfiltriert. Das Salz LiCl wird mit 2 ml Acetonitril gewaschen und die organischen Phasen vereinigt. Das Lösungsmittel wird abdestilliert und der Rückstand im Vakuum bei 7 Pa und 50°C getrocknet.
Man erhält 0,99 g Hexamethylguanidinium Thiocyanat als Öl, das entspricht einer Ausbeute von 94,8 %.
¹H NMR (Referenz: TMS intern; CD₃CN), ppm: 2.89 s (6CH₃).

### Beispiel 14:

### A) Bis(dimethylamino)chlorcarbenium Tetracyanoborat

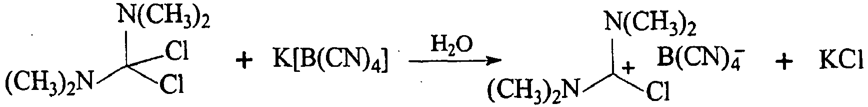

Zu einer Lösung von 12,0 g (77,9 mmol) Kaliumtetracyanoborat in 200 ml Wasser, werden bei Raumtemperatur unter Rühren 13,4 g (78,3 mmol) Bis(dimethylamino)dichlormethan zugegeben. Die Reaktionsmischung wird 10 Minuten gerührt und mit Hilfe eines Eisbades gekühlt. Anschließend wird der Niederschlag abfiltriert und mehrmals mit 30 ml Eiswasser gewaschen. Die vereinigten Filtrate wurden 3 Stunden im Vakuum von 7 Pa und 60°C im Ölbad getrocknet.
Man erhält 13,4 g Bis(dimethylamino)chlorcarbenium Tetracyanoborat als Öl, das entspricht einer Ausbeute von 68,7 %.
¹H NMR (Referenz: TMS intern; CD₃CN), ppm: 3.27 s (4CH₃).
¹¹B NMR (Referenz: BF₃ · Et₂O extern; CD₃CN), ppm: -38.59 s.

### B) N,N,N',N'-Tetramethyl-N",N"-diethylguanidinium Tetracyanoborat

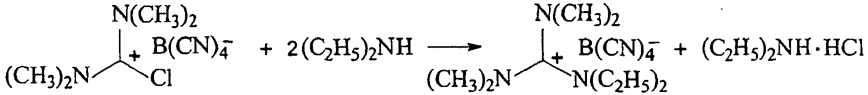

Zu 12,0 g (47,9 mmol) Bis(dimethylamino)chlorcarbenium Tetracyanoborat werden unter Rühren und Kühlung 10,5 g (143,6 mmol) Diethylamin zugegeben. Die Reaktionsmischung wird 3 Stunden bei Raumtemperatur gerührt und danach 30 ml Wasser zugegeben. Die untere Phase wird abgetrennt und es wird mit 30 ml Dichlormethan verdünnt. Die organischen Phasen werden anschließend mehrmals mit 30 ml Wasser gewaschen und mit MgSO₄ getrocknet. Danach wird das Lösungsmittel abdestilliert und der Rückstand 3 Stunden im Vakuum von 7 Pa bei 60°C getrocknet.
Man erhält 13,4 g N,N,N',N'-Tetramethyl-N",N"-diethylguanidinium Tetracyanoborat als Flüssigkeit, das entspricht einer Ausbeute von 97,4 %. ¹H NMR (Referenz: TMS intern; CD₃CN), ppm: 1.15 t (2CH₃), 2.88 s (2CH₃), 2.90 s (2CH₃), 3.23 m (2CH₂), ³J_{H,H} = 7.2 Hz.
¹¹B NMR (Referenz: BF_{3 ·} Et₂O extern; CD₃CN), ppm: -38.58 s.

### Beispiel 15:

### A) 1,3-Dimethyl-2-fluorimidazolidinium Bis(trifluormethyl)imid

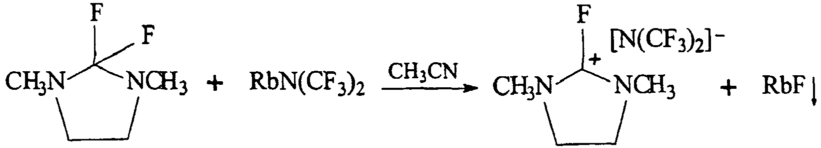

Zu einer Lösung von 0,73 g (3,10 mmol) RbN(CF₃)₂, hergestellt aus der Umsetzung von 0,32 g RbF mit 0,96 g Trifluormethansulfonylfluorid, bekannt aus EP 1081129 B1, in 7 ml trockenem Acetonitril wird eine Lösung von 0,46 g (3,37 mmol) 1,3-Dimethyl-2,2-difluorimidazolidin in 4,57 g trockenem Acetonitril unter Rühren bei Raumtemperatur zugegeben. 1,3-Dimethyl-2,2-difluorimidazolidin wurde analog zu der Beschreibung aus A.A. Kolomeitcev et al., J. of Fluorine Chem. 103 (2000)159-162 durch Reaktion von 1,3-Dimethyl-2,2-dichlorimidazolidin mit KF in Acetonitril erhalten. Die Reaktionsmischung wird 30 Minuten gerührt und anschließend RbF unter Schutzgasatmosphäre abfiltriert und mehrmals mit 3 ml trockenem Acetonitril gewaschen. Das Lösungsmittel wird im Vakuum bei 0°C abdestilliert und getrocknet. Man erhält 1,3-Dimethyl-2-diethylaminoimidazolidinium Bis(trifluormethyl)imid.
¹H NMR (Referenz: TMS intern; CD₃CN), ppm: 2.96 br. s (2CH₃), 3.87 br. s (2CH₂).
¹⁹F NMR (Referenz: CCl₃F intern; CD₃CN), ppm: -36.42 s (2CF₃), -53.74 s (CF).

### B) 1,3-Dimethyl-2-diethylamino-imidazolidinium Bis(trifluormethyl)imid

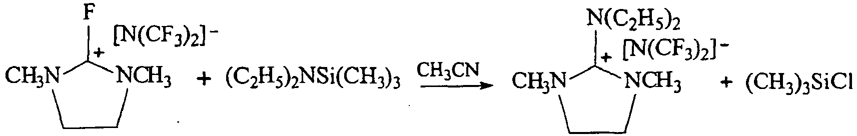

Zu einer Lösung von 0,83 g (3,10 mmol) 1,3-Dimethyl-2-fluorimidazolidinium Bis(trifluormethyl)imid in 10 ml trockenem Acetonitril, werden 0,50 g (3,44 mmol) N,N-Diethyltrimethylsilylamin innerhalb von 5 Minuten unter Eiskühlung zugegeben. Die Reaktionsmischung wird 30 Minuten bei 0°C gerührt und anschließend auf Raumtemperatur gebracht. Die flüchtigen Bestandteile werden im Vakuum abdestilliert und der Rückstand im Vakuum bei 7,0 Pa und Raumtemperatur getrocknet.
Man erhält 0,59 g 1,3-Dimethyl-2-diethylamino-imidazolidinium Bis(trifluormethyl)imid, das entpricht einer Ausbeute von 59,4 %.
¹H NMR (Referenz: TMS intern; CD₃CN), ppm: 1.13 t (2CH₃), 2.91 s (2CH₃), 3.30 q (2CH₂), 3.66 s (2CH₂), ³J_{H,H} = 7.1 Hz.
¹⁹F NMR (Referenz: CCl₃F intern; CD₃CN), ppm: -35.96 s (2CF₃).

### Beispiel 16:

### A) 1,3-Dimethyl-2-chlorimidazolidinium Bis(fluorsulfonyl)imid

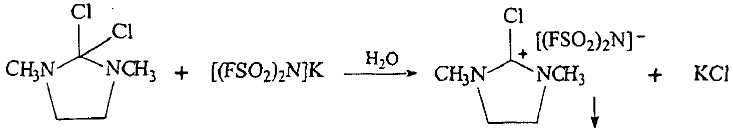

Zu einer Lösung von 3,0 g (17,7 mmol) 1,3-Dimethyl-2,2-dichlorimidazolidin in 15 ml Wasser wird unter Rühren bei Raumtemperatur eine Lösung von 3,9 g (17,8 mmol) Kalium Bis(fluorsulfonyl)imid in 20 ml Wasser zugegeben. Die Reaktionsmischung wird 30 Minuten gerührt und anschließend der Rückstand abfiltriert, mit 20 ml Wasser gewaschen und im Vakuum bei 7 Pa und 60°C getrocknet.
Man erhält 5,15 g 1,3-Dimethyl-2-chlorimidazolidinium Bis(fluorsulfonyl)imid, das entpricht einer Ausbeute von 92,7 %.
Smp.: 129-130°C
¹H NMR (Referenz: TMS intern; CD₃CN), ppm: 3.13 s (2CH₃), 3.94 s (2CH₂).
¹⁹F NMR (Referenz: CCl₃F intern; CD₃CN), ppm: 52.38 s (2SO₂F).

### B) 1,3-Dimethyl-2-diethylamino-imidazolidinium Bis(fluorsulfonyl)imid

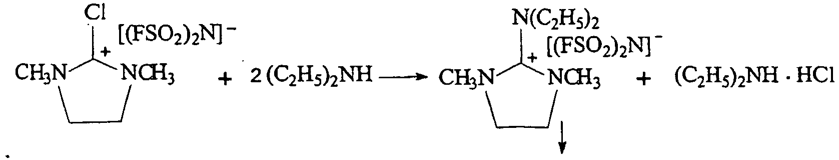

Zu 2,0 g (6,38 mmol) 1,3-Dimethyl-2-chlorimidazolidinium
Bis(fluorsulfonyl)imid werden 1,4 g (19,14 mmol) Diethylamin unter Rühren bei Raumtemperatur zugegeben. Die Reaktionsmischung wird 20 Minuten gerührt und 10 ml Wasser zugegeben. Nach 5 Minuten wird der Rückstand abfiltriert und mehrmals mit 10 ml Wasser gewaschen ung im Vakuum bei 7 Pa und 60°C getrocknet.
Man erhält 2,15 g 1,3-Dimethyl-2-diethylamino-imidazolidinium Bis(fluorsulfonyl)imid, das entpricht einer Ausbeute von 96,2 %.
¹H NMR (Referenz: TMS intern; CD₃CN), ppm: 1.17 t (2CH₃), 2.92 s (2CH₃), 3.32 q (2CH₂), 3.66 s (2CH₂), ³J_{H,H} = 7.1 Hz.
¹⁹F NMR (Referenz: CCl₃F intern; CD₃CN), ppm: 52.38 s (2SO₂F).

### Beispiel 17:

### A) Bis(dimethylamino)chlorcarbenium Methylsulfat

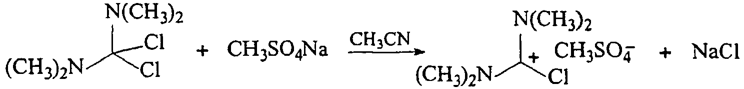

Zu 1.96 g (11.46 mmol) Bis(dimethylamino)dichlormethan werden unter Inertgasatmosphäre 1.54 g (11.48 mmol) Natriummethylsulfat und 10 ml trockenes Acetonitril zugefügt. Die Reaktionsmischung wird 5 Stunden bei Raumtemperatur gerührt und der Niederschlag an NaCl abfiltriert. Das Lösungsmittel des Filtrats wird anschließend abedestilliert und der erhaltene Rückstand wird 1 Stunde bei einer Ölbadtemperatur von 50°C im Vakuum bei 7.0 Pa getrocknet. Man erhält 2.78 g Bis(dimethylamino)chlorcarbenium Methylsulfat als viskose Flüssigkeit, das entspricht einer Ausbeute von 98.3 %.
¹H NMR (Referenz: TMS intern; Acetonitril-D₃), ppm: 3.28 s (4CH₃), 3.62 s (CH₃).

### B) Hexamethylguanidinium Methylsulfat

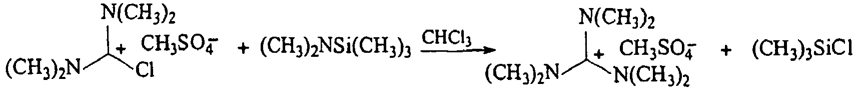

Zu 2.45 g (9.93 mmol) Bis(dimethylamino)chlorcarbenium Methylsulfat werden 10 ml trockenes Chloroform und 1.29 g (11.00 mmol) N,N-Dimethyltrimethylsilylamin zugegeben. Die Reaktionsmischung wird 1 Stunde bei Raumtemperatur gerührt und anschließend alle flüchtigen Verbindungen im Vakuum entfernt. Der Rückstand wird noch 1 Stunde im Vakuum bei 7.0 Pa und 50°C Ölbadtemperatur getrocknet. Man erhält 2.42 g Hexamethylguanidinium Methylsulfat, das entspricht einer Ausbeute von 95.5 %.

### Smp.: 187-188°C.

¹H NMR (Referenz: TMS intern; Acetonitril-D₃), ppm: 2.88 s (6CH₃), 3.51 s (CH₃).

### Beispiel 18:

### A) Bis(dimethylamino)chlorcarbenium Bis(pentafluorethyl)phosphinat

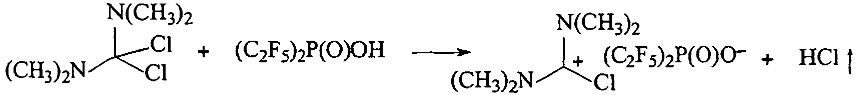

Zu 3.33 g (19.47 mmol) Bis(dimethylamino)dichlormethan werden 5.88 g (19.47 mmol) Bis(pentafluorethyl)phosphinsäure zugegeben. Der entstehende Chlorwasserstoff wird im Stickstoffstrom entfernt. Die Reaktionsmischung wird bei Raumtemperatur 30 Minuten gerührt und der Rückstand im Vakuum bei 7 Pa und 60°C Ölbadtemperatur innerhalb von 3 Stunden getrocknet. Man erhält 8.48 g Bis(dimethylamino)chlorcarbenium Bis(pentafluorethyl)phosphinat als Flüssigkeit, das entspricht einer Ausbeute von 99.7 %.
¹H NMR (Referenz: TMS intern; Acetonitril-D₃), ppm: 3.27 s (4CH₃).
¹⁹F NMR (Referenz: CCl₃F intern; Acetonitril-D₃), ppm: -80.18 s (2CF₃), - 124.88 d (2CF₂), ²J_{P,F} = 68 Hz.
³¹P NMR (Acetonitril-D₃; Standard: 85% H₃PO₄ extern), ppm: -2.64 quin., ²J_{P,F} = 68 Hz.

### B) Hexamethylguanidinium Bis(pentafluorethyl)phosphinat

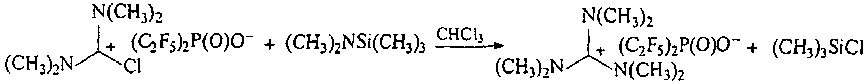

Zu 6.23 g (14.27 mmol) Bis(dimethylamino)chlorcarbenium Bis(pentafluorethyl)phosphinat werden 20 ml trockenes Chloroform und 1.85 g (15.78 mmol) N,N-Dimethyltrimethylsilylamin zugegeben. Die Reaktionsmischung wird 1 Stunde bei Raumtemperatur gerührt und alle flüchtigen Produke im Vakuum entfernt. Der Rückstand wird anschließend bei 7.0 Pa und 50°C Ölbadtemperatur 1 Stunde getrocknet. Man erhält 6.29 g Hexamethylguanidinium Bis(pentafluorethyl)phosphinat, das entspricht einer Ausbeute von 99 %.

Smp.: 45-47°C.
¹H NMR (Referenz: TMS intern; Acetonitril-D₃), ppm: 2.88 s (6CH₃).
¹⁹F NMR (Referenz: CCl₃F intern; Acetonitril-D₃), ppm : -80.21 s (2CF₃), - 124.89 d (2CF₂), ²J_{P,F} = 66 Hz.
³¹P NMR (Acetonitril-D₃; Standard: 85% H₃PO₄ extern), ppm: -2.62 quin., ²J_{P,F} = 66 Hz.

### Beispiel 19:

### A) 1,3-Dimethyl-2-chlorimidazolidinium Methylsulfat

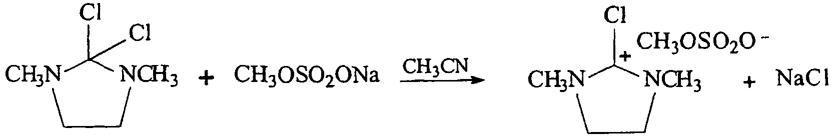

3.00g(17.75 mmol) 1,3-Dimethyl-2,2-dichlorimidazolidin und 2.38 g (17.75 mmol) Natriummethylsulfat werden mit 20 ml trockenem Acetonitril gemischt. Die Reaktionsmischung wird 5 Stunden bei Raumtemperatur gerührt und der Niederschlag NaCl wird abfiltriert. Das Lösungsmittel wird abdestilliert und der Rückstand 1 Stunde im Vakuum von 7 Pa und 50°C getrocknet. Man erhält 4.09 g 1,3-Dimethyl-2-chlorimidazolidinium Methylsulfat als viskoses Material, das entspricht einer Ausbeute von 94.2 %.
¹H NMR (Referenz: TMS intern; CD₃CN), ppm: 3.13 s (2CH₃), 3.50 s (OCH₃), 3.98 s (2CH₂).

### B) 1,3-Dimethyl-2-dimethylamino-imidazolidinium Methylsulfat

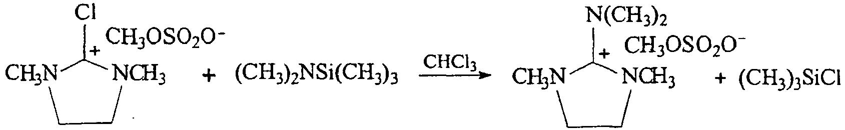

Zu 2.50 g (10.22 mmol) 1,3-Dimethyl-2-chlorimidazolidinium Methylsulfat in 10 ml trockenem Chloroform werden 1.33 g (11.34 mmol) N,N-Diethyltrimethylsilylamin innerhalb von 5 Minuten bei Raumtemperatur und unter starkem Rühren zugegeben. Das Lösungsmittel wird abdestilliert und der Rückstand dreimal mit 5 ml Diethylether gewaschen und anschließend im Vakuum von 7.0 Pa und bei 50°C für 1 Stunde getrocknet. Das Produkt wird aus einer Mischung von Tetrahydrofuran:Diethylether (1:1) kristallisiert. Man erhält 2.22 g 1,3-Dimethyl-2-dimethylamino-imidazolidinium Methylsulfat, das entspricht einer Ausbeute von 85.7 %.

Smp.: 124-127°C.
¹H NMR (Referenz: TMS intern; CD₃CN), ppm: 2.97 s (2CH₃), 2.99 s (2CH₃), 3.49 s (OCH₃), 3.65 s (2CH₂).

### Beispiel 20:

### A) 1,3-Dimethyl-2-chlorimidazolidinium Dihydrophosphat

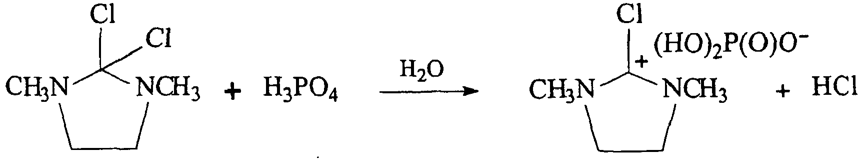

5.0 g (29.58 mmol) 1,3-Dimethyl-2,2-dichlorimidazolidin und 3.41 g einer 85%igen Phosphorsäure (29.58 mmol) werden bei Raumtemperatur gemischt. Die Reaktionsmischung wird für 1 Stunde bei 60°C gerührt. Alle flüchtigen Bestandteile werden im Vakuum bei 7 Pa und 60°C entfernt. Man erhält 6.82 g 1,3-Dimethyl-2-chlorimidazolidinium Dihydrophosphat. Die Ausbeute ist annähernd quantitativ.
¹H NMR (Referenz: TMS intern; D₂O), ppm: 2.89 s (2CH₃), 3.73 s (2CH₂), 4.78 s (OH).
³¹P NMR (D₂O; Standard: 85% H₃PO₄ extern), ppm: 2.4 s.

### B) 1,3-Dimethyl-2-diethylamino-imidazolidinium Dihydrophosphat

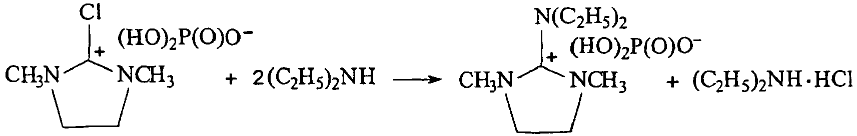

Zu 6.70 g (29.06 mmol) 1,3-Dimethyl-2-chlorimidazolidinium Dihydrophosphat werden 4.26 g (58.24 mmol) Diethylamin und 10 ml Wasser unter Rühren zugegeben. Die Reaktionsmischung wird 10 Minuten weiter gerührt und die Lösung 9 Mal mit 30 ml Dichlormethan extrahiert. Der Extrakt wird mit Magnesiumsulfat getrocknet und das Lösungsmittel abdestilliert. Der Rückstand wird 2 Stunden im Vakuum bei 7 Pa und 60°C Ölbadtemperatur getrocknet. Man erhält 5.58 g 1,3-Dimethyl-2-diethylamino-imidazolidinium Dihydrophosphat, das entspricht einer Ausbeute von 97.6 %.
¹H NMR (Referenz: TMS intern; D₂O), ppm: 0.97 t (2CH₃), 2.76 s (2CH₃), 3.16 q (2CH₂), 3.50 s (2CH₂), 4.73 s (OH), ³J_{H,H} = 7.2 Hz.
³¹P NMR (D₂O; Standard: 85% H₃PO₄ extern), ppm: 2.9 s.

### Beispiel 21:

### A) 1,3-Dimethyl-2-chlorimidazolidinium Dimethylphosphat

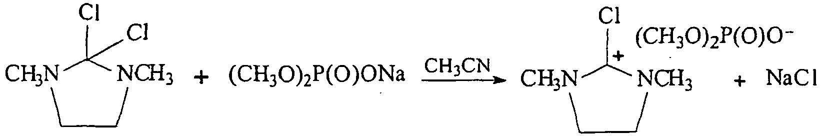

3.00 g (17.75 mmol) 1,3-Dimethyl-2,2-dichlorimidazolidin und 2.63 g (17.77 mmol) Natriumdimethylphosphat werden mit 20 ml Acetonitril und 2 ml Wasser gemischt. Die Reaktionsmischung wird für 12 Stunden bei Raumtemperatur gerührt und anschließend der Niederschlag NaCl abfiltriert. Das Lösungsmittel wird abdestilliert und der Rückstand 6 Stunden im Vakuum von 7 Pa und 70-80°C Ölbadtemperatur getrocknet. Man erhält 4.39 g 1,3-Dimethyl-2-chlorimidazolidinium Dimethylphosphat, das entspricht einer Ausbeute von 95.6 %.
¹H NMR (Referenz: TMS intern; CD₃CN), ppm: 3.10 s (2CH₃), 3.50 d (2OCH₃), 3.94 s (2CH₂), ³J_{H,P} = 10.6 Hz.
³¹P NMR (D₂O; Standard: 85% H₃PO₄ extern), ppm: 7.0 quin., ³J_{H,P} = 10.5 Hz.

### B) 1,3-Dimethyl-2-dimethylamino-imidazolidinium Dimethylphosphat

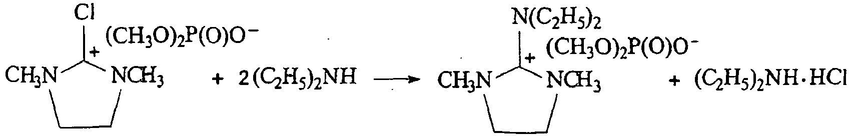

Zu 2.0 g (7.73 mmol) 1,3-Dimethyl-2-chlorimidazolidinium Dimethylphosphat werden 1.14 g (15.59 mmol) Diethylamin und 10 ml Wasser unter Rühren zugegeben. Die Reaktionsmischung wird 10 Minuten bei Raumtemperatur gerührt und anschließend werden 10 ml Ethanol zugegeben. Die Lösung wird 5 Mal mit 30 ml Dichlormethan extrahiert. Aus der wässrigen Phase wird Wasser abdestilliert. Der Rückstand wird 4 Stunden im Vakuum von 7 Pa bei 70-80°C Ölbadtemperatur getrocknet. Man erhält 1.85 g 1,3-Dimethyl-2-dimethylamino-imidazolidinium Dimethylphosphat, das entspricht einer Ausbeute von 81 %.

Smp.: 123-125°C.
¹H NMR (Referenz: TMS intern; CD₃CN) , ppm: 1.10 t (2CH₃), 2.89 s (2CH₃), 3.30 q (2CH₂), 3.50 d (2OCH₃), 3.63 s (2CH₂), ³J_{H,P} = 10.7 Hz, ³J_{H,H} = 7_{.}1 Hz.
³¹P NMR (D₂O ; Standard: 85% H₃PO₄ extern), ppm: 7.0 quin., ³J_{H,P} = 10.6 Hz.

## Patentansprüche

1. Verfahren zur Herstellung von Guanidinium-Salzen der Formel (1) 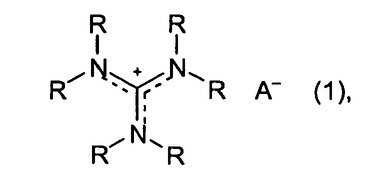 worin die Substituenten R jeweils unabhängig voneinander die Bedeutung von
Wasserstoff,
geradkettiges oder verzweigtes Alkyl mit 1-20 C-Atomen, gesättigtes, teilweise oder vollständig ungesättigtes Cycloalkyl mit 3-7 C-Atomen, welches mit Alkylgruppen mit 1-6 C-Atomen substituiert sein kann, haben,
wobei ein oder mehrere Substituenten R teilweise oder vollständig durch Halogen oder teilweise durch CN oder NO₂ substituiert sein können und Halogen F, Cl, Br oder I bedeutet,
wobei bis zu vier Substituenten R paarweise durch Einfach- oder Doppelbindung miteinander verbunden sein können
und wobei ein Kohlenstoffatom oder zwei nicht benachbarte Kohlenstoffatome eines oder mehrerer Substituenten R durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet
und
A- ein Sulfonat, Alkyl- oder Aryl-Sulfat, Hydrogensulfat, Imid, Methanid, Carboxylat, Phosphat, Phosphinat, Phosphonat, Borat, Thiocyanat, Perchlorat, Fluorsilikat oder Nitrat ist,
durch Umsetzung einer Verbindung der Formel (2) 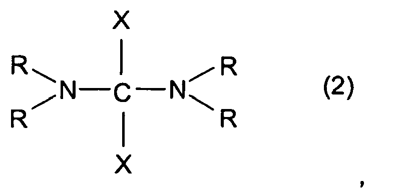 worin die Substituenten R eine bei Formel (1) angegebene Bedeutung haben und X F, Cl oder Br bedeutet,
mit einer Verbindung der Formel (3)
Kt⁺ A- (3),
worin A- eine bei Formel (1) angegebene Bedeutung hat und
Kt⁺ ein Proton, R"₃Si, Alkali- oder Erdalkalimetallkation, Ammoniumkation, Phosphoniumkation oder ein Kation der Gruppe 11 oder 12 sein kann,
wobei R" jeweils unabhängig voneinander Phenyl oder eine lineare oder verzweigte Alkylgruppe mit 1-6 C-Atomen, die durch Phenyl substituiert sein kann, bedeutet,
und anschließender Reaktion der erhaltenen Verbindung der Formel (4) 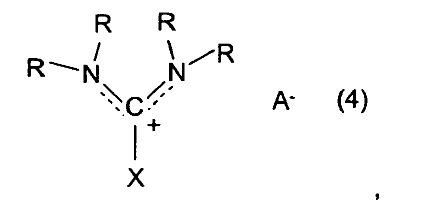 wobei die Substituenten R, X und A⁻ eine bei Formel (1) oder (2) angegebene Bedeutung haben,
mit Verbindungen der Formel (5) 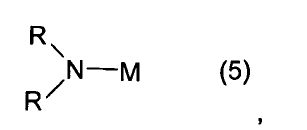 wobei die Substituenten R eine bei Formel (1) angegebene Bedeutung haben und
M Wasserstoff, ein Alkali- oder Erdalkalimetall bedeutet.

2. Verfahren nach Anspruch 1, **dadurch gekennzeichnet, dass** Verbindungen der Formel Kt⁺ A⁻ (3) eingesetzt werden, worin Kt⁺ eine bei Anspruch 1 angegebene Bedeutung hat und
A- aus der Gruppe
[R¹OSO_{3]}⁻, [R¹SO₃]⁻, [R^{F}SO₃]⁻, [(FSO₂)₂N]⁻, [(R^{F}SO₂)₂N]⁻, [(R^{F}SO₂)(R^{F}CO)N]⁻, [(R^{F}SO₂)₃C]⁻, [(FSO₂)₃C]⁻, [R¹CH₂C(O)O]⁻, [R^{F}C(O)O]⁻, [P(CₙF₂ₙ₊₁₋ₘHₘ)_{y}F_{6-y}]⁻, [P(C₆F₅)_{y}F_{6-y}]⁻, [(R¹O)₂P(O)O]⁻, (R¹₂P(O)O]⁻, [R¹P(O)O₂]²⁻, [R^{F}₂P(O)O]⁻, [R^{F}P(O)O₂]²⁻, [BF_{4-z}R^{F}_{z}]⁻, [BF_{4-z}(CN)_{z}]⁻, [B(C₆F₅)₄]⁻, [B(OR¹)₄]⁻, [N(CN)₂] ⁻, [C(CN)₃]⁻, [N(CF₃)₂]-, [HSO₄]⁻, [SiF₆]²⁻, [ClO₄]⁻, [SCN]⁻ und [NO₃]⁻ ausgewählt wird,
worin die Substituenten R^{F} jeweils unabhängig voneinander die Bedeutung von
perfluoriertes und geradkettiges oder verzweigtes Alkyl mit 1-20 C-Atomen,
perfluoriertes und geradkettiges oder verzweigtes Alkenyl mit 2-20 C-Atomen und einer oder mehreren Doppelbindungen,
perfluoriertes und gesättigtes, teilweise oder vollständig ungesättigtes Cycloalkyl mit 3-7 C-Atomen, das mit Perfluoralkylgruppen substituiert sein kann,
wobei die Substituenten R^{F} paarweise durch Einfach- oder Doppelbindung miteinander verbunden sein können und
wobei ein Kohlenstoffatom oder zwei nicht benachbarte Kohlenstoffatome des Substituenten R^{F}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-, - NR'-, -PR'- und -P(O)R'- ersetzt sein können, wobei R' nicht fluoriertes, teilweise oder pertluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet,
worin die Substituenten R¹ jeweils unabhängig voneinander die Bedeutung von
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann,
wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, - SO₃-, -N=, -N=N-, -NH-, -NR'-, -PR'-, -P(O)R'-, P(O)R'O-, OP(O)R'O-, - PR'₂=N-, -C(O)NH-, -C(O)NR'-, -SO₂NH- oder -SO₂NR'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet und die Variablen
n 1 bis 20,
m 0, 1, 2 oder 3,
y 0, 1, 2, 3 oder 4 und
z 0, 1, 2, 3 oder 4 bedeuten.

3. Verfahren nach Anspruch 1 oder 2, **dadurch gekennzeichnet, dass** A⁻ aus der Gruppe
[CH₃OSO₃]⁻, [C₂H₅OSO₃]⁻, [C(CN)₃]⁻,
[CH₃SO₃]⁻, [C₈H₁₇SO₃]⁻, [CH₃C₆H₄SO₃]⁻, [CF₃SO₃]⁻, [C₂H₅SO₃]⁻,
[CF₃CF₂SO₃]⁻, [(CF₃SO₂)₂N]⁻, [(FSO₂)₂N]⁻, [(CF₃SO₂)(CF₃CO)N]⁻,
[(C₂F₅SO₂)(CF₃CO)N]⁻, [(C₂F₅SO₂)₂N]⁻, [(CF₃SO₂)₃C]⁻, [(C₂F₅SO₂)₃C]⁻,
[(FSO₂)₃C]⁻, [CH₃C(O)O]⁻, [C₂H₅C(O)O]⁻, [CF₃C(O)O]⁻,
[CF₃CF₂C(O)O]⁻, [PF₆]⁻, [P(C₂F₅)₃F₃]⁻, [P(C₄F₉)₃F₃]⁻, [P(CF₃)₃F₃]⁻,
[P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻,
[P(C₃F₇)₃F₃]⁻, [P(C₂F₅)₂F₄]⁻, [(HO)₂P(O)O]⁻, [(CH₃O)₂P(O)O]⁻,
[(C₂H₅O)₂P(O)O]⁻, [(C₂F₅)₂P(O)O]⁻, [(C₂F₅)P(O)O₂]²⁻, [P(C₆F₅)₂F₄]⁻,
[(CH₃)₂P(O)O]⁻, [CH₃P(O)O₂]²⁻, [(CF₃)₂P(O)O]⁻, [CF₃P(O)O₂]²⁻, [BF₄]⁻,
[BF₃(CF₃)]⁻, [BF₂(C₂F₅)₂]⁻, [BF₃(C₂F₅)]⁻, [BF₂(CF₃)₂]⁻, [B(C₂F₅)₄]⁻_{,}
[BF₃(CN)]⁻, [BF₂(CN)₂]⁻, [B(CN)₄]⁻, [B(OCH₃)₄]⁻, [B(CF₃)₄]⁻,
[B(OCH₃)₂(OC₂H₅)₂]⁻, [B(O₂C₂H₄)₂]⁻, [B(O₂C₂H₂)₂]⁻, [B(O₂C₆H₄)₂]⁻,
[N(CN)₂]⁻, [N(CF₃)₂]⁻, [HSO₄]⁻, [ClO₄]⁻, [SiF₆]⁻, [SCN]⁻ oder [NO₃]⁻ ausgewählt wird.

4. Verfahren nach einem oder mehreren der Ansprüche 1 bis 3, **dadurch gekennzeichnet, dass** in Dihalogenverbindungen der Formel (2) nach Anspruch 1 der Substituent X Fluor oder Chlor bedeutet.

5. Verfahren nach einem oder mehreren der Ansprüche 1 bis 4, **dadurch gekennzeichnet, dass** in Verbindungen der Formel (5) nach Anspruch 1 der Substituent R jeweils unabhängig voneinander die Bedeutung von Wasserstoff,
geradkettiges oder verzweigtes Alkyl mit 1-20 C-Atomen oder gesättigtes, teilweise oder vollständig ungesättigtes Cycloalkyl mit 3-7 C-Atomen, welches mit Alkylgruppen mit 1-6 C-Atomen substituiert sein kann, hat.

6. Verfahren nach einem oder mehreren der Ansprüche 1 bis 5, **dadurch gekennzeichnet, dass** der erste Schritt des Verfahrens in Wasser durchgeführt wird.

7. Verfahren nach einem oder mehreren der Ansprüche 1 bis 6, **dadurch gekennzeichnet, dass** der erste Schritt des Verfahrens bei Temperaturen von 0° bis 150°C durchgeführt wird.

8. Verfahren nach einem oder mehreren der Ansprüche 1 bis 5, **dadurch gekennzeichnet, dass** die erste Stufe des Verfahrens in einem organischen Lösungsmittel durchgeführt wird.

9. Verfahren gemäß einem oder mehreren der Ansprüche 1 bis 5 und 8, **dadurch gekennzeichnet, dass** der erste Schritt des Verfahrens bei Temperaturen von -50° bis 150°C durchgeführt wird.

10. Verfahren nach einem oder mehreren der Ansprüche 1 bis 9, **dadurch gekennzeichnet, dass** der zweite Schritt des Verfahrens ohne Lösungsmittel durchgeführt wird.

11. Verfahren nach einem oder mehreren der Ansprüche 1 bis 10, **dadurch gekennzeichnet, dass** der zweite Schritt des Verfahrens bei einer Temperatur durchgeführt wird, bei der mindestens eine Komponente flüssig ist.

12. Verfahren nach einem oder mehreren der Ansprüche 1 bis 9, **dadurch gekennzeichnet, dass** der zweite Schritt des Verfahrens in einem organischen Lösungsmittel durchgeführt wird.

13. Verfahren nach einem oder mehreren der Ansprüche 1 bis 9 und 12, **dadurch gekennzeichnet, dass** der zweite Schritt des Verfahrens bei Temperaturen von -50° bis 150°C durchgeführt wird.

14. Verfahren nach einem oder mehreren der Ansprüche 1 bis 9, **dadurch gekennzeichnet, dass** der zweite Schritt des Verfahrens in Wasser durchgeführt wird.

15. Verfahren nach einem oder mehreren der Ansprüche 1 bis 9 und 14, **dadurch gekennzeichnet, dass** der zweite Schritt des Verfahrens bei Temperaturen von 0° bis 150°C durchgeführt wird.

16. Verbindungen der Formel (4), 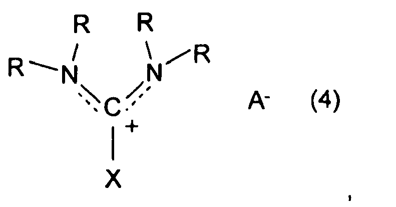 worin die Substituenten R jeweils unabhängig voneinander die Bedeutung von
Wasserstoff,
geradkettiges oder verzweigtes Alkyl mit 1-20 C-Atomen, gesättigtes, teilweise oder vollständig ungesättigtes Cycloalkyl mit 3-7 C-Atomen, welches mit Alkylgruppen mit 1-6 C-Atomen substituiert sein kann, haben,
wobei ein oder mehrere Substituenten R teilweise oder vollständig durch Halogen oder teilweise durch CN oder NO₂ substituiert sein können und Halogen F, Cl, Br oder I bedeutet,
wobei bis zu vier Substituenten R paarweise durch Einfach- oder Doppelbindung miteinander verbunden sein können und wobei ein Kohlenstoffatom oder zwei nicht benachbarte Kohlenstoffatome eines oder mehrerer Substituenten R durch Atome und/oder Atomgruppierungen, ausgewählt aus der Gruppe -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O-, und -P(R')₂=N- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet,
X F, Cl oder Br bedeutet
mit der Maßgabe, dass nicht alle vier Substituenten R gleichzeitig Wasserstoff sind und
A⁻ aus der Gruppe
[R¹OSO₃]⁻, [R¹SO₃]⁻, [R^{F}SO₃]⁻, [(FSO₂)₂N]⁻, [(R^{F}SO₂)(R^{F}CO)N]⁻, [(R^{F}SO₂)₃C]⁻, [(FSO₂)₃C]⁻, [R¹CH₂C(O)O]⁻, [P(CₙF₂ₙ₊₁₋ₘHₘ)_{y}F_{6-y}]⁻, [P(C₆F₅)_{y}F_{6-y}]⁻, [(R¹O)₂P(O)O]⁻, [R¹₂P(O)O]⁻, [R¹P(O)O₂]²⁻, [R^{F}₂P(O)O]⁻, [R^{F}P(O)O₂]²⁻, [BF_{4-z}R^{F}z]⁻, [BF_{4-z}(CN)_{z}]⁻, [B(C₆F₅)₄]⁻, [B(OR¹)₄]⁻, [N(CN)₂]⁻, [(CN₃)C]⁻, [N(CF₃)₂]⁻, [SiF₆]²⁻ und [SCN]⁻ ausgewählt wird, wobei [CF₃SO₃]⁻ ausgenommen ist und
worin die Substituenten R^{F} jeweils unabhängig voneinander die Bedeutung von
perfluoriertes und geradkettiges oder verzweigtes Alkyl mit 1-20 C-Atomen,
perfluoriertes und geradkettiges oder verzweigtes Alkenyl mit 2-20 C-Atomen und einer oder mehreren Doppelbindungen,
perfluoriertes und gesättigtes, teilweise oder vollständig ungesättigtes Cycloalkyl mit 3-7 C-Atomen, das mit Perfluoralkylgruppen substituiert sein kann,
wobei die Substituenten R^{F} paarweise durch Einfach- oder Doppelbindung miteinander verbunden sein können und
wobei ein Kohlenstoffatom oder zwei nicht benachbarte Kohlenstoffatome des Substituenten R^{F}, die nicht α-ständig zu dem Heteroatom stehen, durch Atome und/oder Atomgruppierungen, ausgewählt aus der Gruppe -O-, -C(O)-, -S-, -S(O)-, -SO₂-, -N=, -N=N-, - NR'-, -PR'- und -P(O)R'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet,
worin die Substituenten R¹ jeweils unabhängig voneinander die
Bedeutung von
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das durch Alkylgruppen mit 1-6 C-Atomen substituiert sein kann,
wobei die Substituenten R¹ teilweise durch CN, NO₂ oder Halogen substituiert sein können und
Halogen F, Cl, Br oder I bedeutet,
wobei die Substituenten R¹ paarweise durch Einfach- oder Doppelbindung miteinander verbunden sein können und
wobei ein Kohlenstoffatom oder zwei nicht benachbarte Kohlenstoffatome des Substituenten R¹, die nicht α-ständig zum Heteroatom stehen, durch Atome und/oder Atomgruppierungen, ausgewählt aus der Gruppe -O-, -C(O)-, -C(O)O-, -S-, -S(O)-, -SO₂-, - SO₃-, -N=, -N=N-, -NH-, -NR'-, -PR'-, -P(O)R'-, P(O)R'O-, OP(O)R'O-, - PR'₂=N-, -C(O)NH-, -C(O)NR'-, -SO₂NH- oder -SO₂NR'- ersetzt sein können, wobei R' nicht fluoriertes, teilweise oder perfluoriertes Alkyl mit 1-6 C-Atomen, gesättigtes oder teilweise ungesättigtes Cycloalkyl mit 3-7 C-Atomen, unsubstituiertes oder substituiertes Phenyl oder unsubstituierter oder substituierter Heterocyclus bedeutet und die Variablen
n 1 bis 20,
m 0, 1, 2 oder 3,
y 1, 2, 3 oder 4 und
z 1, 2, 3 oder 4 bedeuten.

17. Verbindungen nach Anspruch 16, **dadurch gekennzeichnet, dass** die Substituenten R Wasserstoff oder eine geradkettige oder verzweigte Alkylgruppe mit 1-12 C-Atomen bedeuten,
mit der Maßgabe, dass nicht alle vier Substituenten R Wasserstoff sind oder mindestens zwei Substituenten R miteinander durch Einfach- oder Doppelbindungen miteinander verbunden sind, so dass ein monocyclisches Kation entsteht und
das Gegenanion A⁻
[CH₃OSO₃]⁻, [C₂H₅OSO₃]⁻, [C(CN)₃]⁻,
[CH₃SO₃]⁻, [C₅H₁₇SO₃]⁻, [CH₃C₆H₄SO₃]⁻, [C₂H₅SO₃]⁻, [CF₃CF₂SO₃]⁻,
[(FSO₂)₂N]⁻, [(CF₃SO₂)(CF₃CO)N]⁻, [(C₂F₅SO₂)(CF₃CO)N]⁻,
[(CF₃SO₂)₃C]⁻, [(C₂F₅SO₂)₃C]⁻, [(FSO₂)₃C]⁻, [CH₃C(O)O]⁻,
[C₂H₅C(O)O]⁻, [P(C₂F₅)₃F₃]⁻, [P(C₄F₉F₃]⁻, [P(CF₃)₃F₃]⁻,
[P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻,
[P(C₃F₇)F₃]⁻, [P(C₂F₅)₂F₄]⁻, [(HO)₂P(O)O]⁻, [(CH₃O)₂P(O)O]⁻,
[(C₂H₅O)₂P(O)O]⁻, [(C₂F₅)₂P(O)O]⁻, [(C₂F₅)P(O)O₂]²⁻, [P(C₆F₅)₂F₄]⁻,
[(CH₃)₂P(O)O]⁻, [CH₃P(O)O₂]²⁻, [(CF₃)₂P(O)O]⁻, [CF₃P(O)O₂]²⁻,
[BF₃(CF₃)]⁻ , [BF₂(C₂F₅)₂]⁻, [BF₃(C₂F₅)]⁻, [BF₂(CF₃)₂]⁻, [B(C₂F₅)₄]⁻,
[BF₃(CN)]⁻, [BF₂(CN)₂]⁻, [B(CN)₄]⁻, [B(OCH₃)₄]⁻, [B(CF₃)4]⁻,
[B(OCH₃)₂(OC₂H₅)₂]⁻, [B(O₂C₂H₄)₂]⁻, [B(O₂C₂H₂)₂]⁻, [B(O₂C₆H₄)₂]⁻,
[N(CN)₂]⁻, [N(CF₃)₂]⁻, [SiF₆]⁻ oder [SCN]⁻ bedeutet.

## Claims

1. Process for the preparation of guanidinium salts of the formula (1) 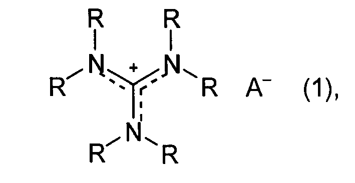 in which the substituents R in each case, independently of one another, have the meaning of
hydrogen,
straight-chain or branched alkyl having 1-20 C atoms, saturated, partially or fully unsaturated cycloalkyl having 3-7 C atoms, which may be substituted by alkyl groups having 1-6 C atoms,
where one or more substituents R may be partially or fully substituted by halogen or partially by CN or NO₂ and halogen denotes F, Cl, Br or I, where up to four substituents R may be connected to one another in pairs by a single or double bond
and where a carbon atom or two non-adjacent carbon atoms of one or more substituents R may be replaced by atoms and/or atom groups selected from the group -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- and -P(R')₂=N-, where R' denotes unfluorinated, partially fluorinated or perfluorinated alkyl having 1-6 C atoms, saturated or partially unsaturated cycloalkyl having 3-7 C atoms, unsubstituted or substituted phenyl or an unsubstituted or substituted heterocycle
and
A⁻ is a sulfonate, alkyl- or arylsulfate, hydrogensulfate, imide, methanide, carboxylate, phosphate, phosphinate, phosphonate, borate, thiocyanate, perchlorate, fluorosilicate or nitrate,
by reaction of a compound of the formula (2) 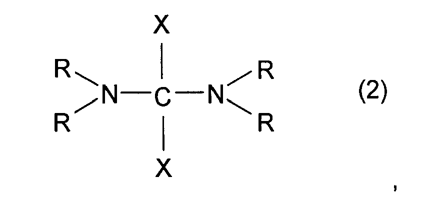 in which the substituents R have a meaning indicated in the case of formula (1) and X denotes F, Cl or Br,
with a compound of the formula (3)
Kt⁺ A⁻ (3),
in which A⁻ has a meaning indicated in the case of formula (1) and Kt⁺ can be a proton, R"₃Si, alkali or alkaline-earth metal cation, ammonium cation, phosphonium cation or a cation from group 11 or 12, where R" in each case, independently of one another, denotes phenyl or a linear or branched alkyl group having 1-6 C atoms, which may be substituted by phenyl,
and subsequent reaction of the resultant compound of the formula (4) 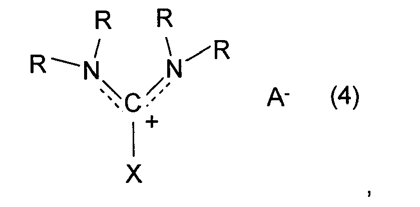 where the substituents R, X and A⁻ have a meaning indicated in the case of formula (1) or (2),
with compounds of the formula (5) 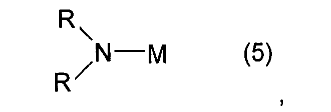 where the substituents R have a meaning indicated in the case of formula (1) and
M denotes hydrogen, an alkali metal or alkaline-earth metal.

2. Process according to Claim 1, **characterised in that** compounds of the formula Kt⁺ A⁻ (3) are employed in which Kt⁺ has a meaning indicated in the case of Claim 1 and
A⁻ is selected from the group
[R¹OSO₃]⁻, [R¹SO₃]⁻, [R^{F}SO₃]⁻, [(FSO₂)₂N]⁻, [(R^{F}SO₂)₂N]⁻, [(R^{F}SO₂)(R^{F}CO)N]⁻, [(R^{F}SO₂)₃C]⁻, [(FSO₂)₃C]⁻, [R¹CH₂C(O)O]⁻, [R^{F}C(O)O]⁻, [P(CₙF₂ₙ₊₁₋ₘHₘ)_{y}F_{6-y}]⁻, [P(C₆F₅)_{y}F_{6-y}]⁻, [(R¹O)₂P(O)O]⁻, [R¹₂P(O)O]⁻, [R¹P(O)O₂]²⁻, [R^{F}₂P(O)O]⁻, [R^{F}P(O)O₂]²⁻, [BF_{4-z}R^{F}_{z}]⁻, [BF_{4-z}(CN)_{z}]⁻, [B(C₆F₅)₄]⁻, [B(OR¹)₄]⁻, [N(CN)₂]⁻, [C(CN)₃]⁻, [N(CF₃)₂]⁻, [HSO₄]⁻, [SiF₆]²⁻, [ClO₄]⁻, [SCN]⁻ and [NO₃]⁻,
in which the substituents R^{F} in each case, independently of one another, have the meaning of
perfluorinated and straight-chain or branched alkyl having 1-20 C atoms, perfluorinated and straight-chain or branched alkenyl having 2-20 C atoms and one or more double bonds,
perfluorinated and saturated, partially or fully unsaturated cycloalkyl having 3-7 C atoms, which may be substituted by perfluoroalkyl groups, where the substituents R^{F} may be connected to one another in pairs by a single or double bond and
where a carbon atom or two non-adjacent carbon atoms of the substituent R^{F} which are not in the α-position to the heteroatom may be replaced by atoms and/or atom groups selected from the group -O-, -C(O)-, -S-, -S(O)-, -SO₂-, -N=, -N=N-, -NR'-, -PR'- and -P(O)R'-, where R' denotes unfluorinated, partially fluorinated or perfluorinated alkyl having 1-6 C atoms, saturated or partially unsaturated cycloalkyl having 3-7 C atoms, unsubstituted or substituted phenyl or an unsubstituted or substituted heterocycle,
in which the substituents R¹ in each case, independently of one another, have the meaning of
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where the substituents R¹ may be partially substituted by CN, NO₂ or halogen and
halogen denotes F, Cl, Br or I,
where the substituents R¹ may be connected to one another in pairs by a single or double bond and
where a carbon atom or two non-adjacent carbon atoms of the substituent R¹ which are not in the α-position to the heteroatom may be replaced by atoms and/or atom groups selected from the group -O-, -C(O)-, -C(O)O-, -S-, -S(O)-, -SO₂-, -SO₃-, -N=, -N=N-, -NH-, -NR'-, -PR'-, -P(O)R'-, P(O)R'O-, OP(O)R'O-, -PR'₂=N-, -C(O)NH-, -C(O)NR'-, -SO₂NH- or -SO₂NR'-, where R' denotes unfluorinated, partially fluorinated or perfluorinated alkyl having 1-6 C atoms, saturated or partially unsaturated cycloalkyl having 3-7 C atoms, unsubstituted or substituted phenyl or an unsubstituted or substituted heterocycle and the variables
n denotes 1 to 20,
m denotes 0, 1, 2 or 3,
y denotes 0, 1, 2, 3 or 4 and
z denotes 0, 1, 2, 3 or 4.

3. Process according to Claim 1 or 2, **characterised in that** A- is selected from the group
[CH₃OSO₃]⁻, [C₂H₅OSO₃]⁻, [C(CN)₃]⁻,
[CH₃SO₃]⁻, [C₈H₁₇SO₃]⁻, [CH₃C₆H₄SO₃]⁻, [CF₃SO₃]⁻, [C₂H₅SO₃]⁻, [CF₃CF₂SO₃]⁻, [(CF₃SO₂)₂N]⁻, [(FSO₂)₂N]⁻, [(CF₃SO₂)(CF₃CO)N]⁻, [(C₂F₅SO₂)(CF₃CO)N]⁻, [(C₂F₅SO₂)₂N]⁻, [(CF₃SO₂)₃C]⁻, [(C₂F₅SO₂)₃C]⁻, [(FSO₂)₃C]⁻, [CH₃C(O)O]⁻, [C₂H₅C(O)O]⁻, [CF₃C(O)O]⁻, [CF₃CF₂C(O)O]⁻, [PF₆]⁻, [P(C₂F₅)₃F₃]⁻, [P(C₄F₉)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻, [P(C₃F₇)₃F₃]⁻, [P(C₂F₅)₂F₄]⁻, [(HO)₂P(O)O]⁻, [(CH₃O)₂P(O)O]⁻, [(C₂H₅O)₂P(O)O]⁻, [(C₂F₅)₂P(O)O]⁻, [(C₂F₅)P(O)O₂]²⁻, [P(C₆F₅)₂F₄]⁻, [(CH₃)₂P(O)O]⁻, [CH₃P(O)O₂]²⁻, [(CF₃)₂P(O)O]⁻, [CF₃P(O)O₂]²⁻, [BF₄]⁻, [BF₃(CF₃)]⁻, [BF₂(C₂F₅)₂]⁻, [BF₃(C₂F₅)]⁻, [BF₂(CF₃)₂]⁻, [B(C₂F₅)₄]⁻, [BF₃(CN)]⁻, [BF₂(CN)₂]⁻, [B(CN)₄]⁻, [B(OCH₃)₄]⁻, [B(CF₃)₄]⁻, [B(OCH₃)₂(OC₂H₅)₂]⁻, [B(O₂C₂H₄)₂]⁻, [B(O₂C₂H₂)₂]⁻, [B(O₂C₆H₄)₂]⁻, [N(CN)₂]⁻, [N(CF₃)₂]⁻, [HSO₄]⁻, [ClO₄]⁻, [SiF₆]⁻, [SCN]⁻ or [NO₃]⁻.

4. Process according to one or more of Claims 1 to 3, **characterised in that** the substituent X in dihalogen compounds of the formula (2) according to Claim 1 denotes fluorine or chlorine.

5. Process according to one or more of Claims 1 to 4, **characterised in that** the substituent R in compounds of the formula (5) according to Claim 1 in each case, independently of one another, has the meaning of hydrogen,
straight-chain or branched alkyl having 1-20 C atoms or
saturated, partially or fully unsaturated cycloalkyl having 3-7 C atoms, which may be substituted by alkyl groups having 1-6 C atoms.

6. Process according to one or more of Claims 1 to 5, **characterised in that** the first step of the process is carried out in water.

7. Process according to one or more of Claims 1 to 6, **characterised in that** the first step of the process is carried out at temperatures of 0° to 150°C.

8. Process according to one or more of Claims 1 to 5, **characterised in that** the first step of the process is carried out in an organic solvent.

9. Process according to one or more of Claims 1 to 5 and 8, **characterised in that** the first step of the process is carried out at temperatures of -50° to 150°C.

10. Process according to one or more of Claims 1 to 9, **characterised in that** the second step of the process is carried out without a solvent.

11. Process according to one or more of Claims 1 to 10, **characterised in that** the second step of the process is carried out at a temperature at which at least one component is liquid.

12. Process according to one or more of Claims 1 to 9, **characterised in that** the second step of the process is carried out in an organic solvent.

13. Process according to one or more of Claims 1 to 9 and 12, **characterised in that** the second step of the process is carried out at temperatures of -50° to 150°C.

14. Process according to one or more of Claims 1 to 9, **characterised in that** the second step of the process is carried out in water.

15. Process according to one or more of Claims 1 to 9 and 14, **characterised in that** the second step of the process is carried out at temperatures of 0° to 150°C.

16. Compounds of the formula (4) 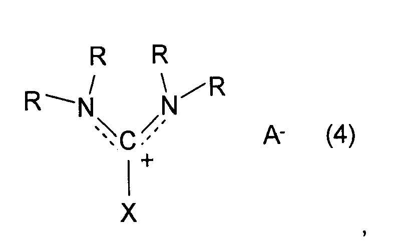 in which the substituents R in each case, independently of one another, have the meaning of
hydrogen,
straight-chain or branched alkyl having 1-20 C atoms, saturated, partially or fully unsaturated cycloalkyl having 3-7 C atoms, which may be substituted by alkyl groups having 1-6 C atoms,
where one or more substituents R may be partially or fully substituted by halogen or partially by CN or NO₂ and
halogen denotes F, Cl, Br or I,
where up to four substituents R may be connected to one another in pairs by a single or double bond
and where a carbon atom or two non-adjacent carbon atoms of one or more substituents R may be replaced by atoms and/or atom groups selected from the group -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O-, and -P(R')₂=N-, where R' denotes unfluorinated, partially fluorinated or perfluorinated alkyl having 1-6 C atoms, saturated or partially unsaturated cycloalkyl having 3-7 C atoms, unsubstituted or substituted phenyl or an unsubstituted or substituted heterocycle,
X denotes F, Cl or Br,
with the proviso that all four substituents R are not simultaneously hydrogen and
A⁻ is selected from the group
[R¹OSO₃]⁻, [R¹SO₃]⁻, [R^{F}SO₃]⁻, [(FSO₂)₂N]⁻, [(R^{F}SO₂)(R^{F}CO)N]⁻, [(R^{F}SO₂)₃C]⁻, [(FSO₂)₃C]⁻, [R¹CH₂C(O)O]⁻, [P(CₙF₂ₙ₊₁₋ₘHₘ)_{y}F_{6-y}]⁻, [P(C₆F₅)_{y}F_{6-y}]⁻, [(R¹O)₂P(O)O]⁻, [R¹₂P(O)O]⁻, [R¹P(O)O₂]²⁻, [R^{F}2P(O)O]⁻,
[R^{F}P(O)O₂]²⁻, [BF_{4-z}R^{F}_{z}]⁻, [BF_{4-z}(CN)_{z}]⁻, [B(C₆F₅)₄]⁻, [B(OR¹)₄]⁻, [N(CN)₂] ⁻, [(CN₃)C]⁻, [N(CF₃)₂]⁻, [SiF₆]²⁻ and [SCN]⁻,
where [CF₃SO₃]⁻ is excluded and
in which the substituents R^{F} in each case, independently of one another, have the meaning of
perfluorinated and straight-chain or branched alkyl having 1-20 C atoms, perfluorinated and straight-chain or branched alkenyl having 2-20 C atoms and one or more double bonds,
perfluorinated and saturated, partially or fully unsaturated cycloalkyl having 3-7 C atoms, which may be substituted by perfluoroalkyl groups, where the substituents R^{F} may be connected to one another in pairs by a single or double bond and
where a carbon atom or two non-adjacent carbon atoms of the substituent R^{F} which are not in the α-position to the heteroatom may be replaced by atoms and/or atom groups selected from the group -O-, -C(O)-, -S-, -S(O)-, -SO₂-, -N=, -N=N-, -NR'-, -PR'- and -P(O)R'-, where R' denotes unfluorinated, partially fluorinated or perfluorinated alkyl having 1-6 C atoms, saturated or partially unsaturated cycloalkyl having 3-7 C atoms, unsubstituted or substituted phenyl or an unsubstituted or substituted heterocycle,
in which the substituents R¹ in each case, independently of one another, have the meaning of
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where the substituents R¹ may be partially substituted by CN, NO₂ or halogen and
halogen denotes F, Cl, Br or I,
where the substituents R¹ may be connected to one another in pairs by a single or double bond and
where a carbon atom or two non-adjacent carbon atoms of the substituent R¹ which are not in the α-position to the heteroatom may be replaced by atoms and/or atom groups selected from the group -O-, -C(O)-, -C(O)O-, -S-, -S(O)-, -SO₂-, -SO₃-, -N=, -N=N-, -NH-, -NR'-, -PR'-, -P(O)R'-, P(O)R'O-, OP(O)R'O-, -PR'₂=N-, -C(O)NH-, -C(O)NR'-, -SO₂NH- or -SO₂NR'-, where R' denotes unfluorinated, partially fluorinated or perfluorinated alkyl having 1-6 C atoms, saturated or partially unsaturated cycloalkyl having 3-7 C atoms, unsubstituted or substituted phenyl or an unsubstituted or substituted heterocycle and the variables
n denotes 1 to 20,
m denotes 0, 1, 2 or 3,
y denotes 1, 2, 3 or 4 and
z denotes 1, 2, 3 or 4.

17. Compounds according to Claim 16, **characterised in that** the substituents R denote hydrogen or a straight-chain or branched alkyl group having 1-12 C atoms,
with the proviso that all four substituents R are not hydrogen or at least two substituents R are connected to one another by single or double bonds to form a monocyclic cation and
the counteranion A⁻ denotes
[CH₃OSO₃]⁻, [C₂H₅OSO₃]⁻, [C(CN)₃]⁻,
[CH₃SO₃]⁻, [C₈H₁₇SO₃]⁻, [CH₃C₆H₄SO₃]⁻, [C₂H₅SO₃]⁻, [CF₃CF₂SO₃]⁻,
[(FSO₂)₂N]⁻, [(CF₃SO₂)(CF₃CO)N]⁻, [(C₂F₅SO₂)(CF₃CO)N]⁻,
[(CF₃SO₂)₃C]⁻, [(C₂F₅SO₂)₃C]⁻, [(FSO₂)₃C]⁻, [CH₃C(O)O]⁻,
[C₂H₅C(O)O]⁻, [P(C₂F₅)₃F₃]⁻, [P(C₄F₉)₃F₃]⁻, [P(CF₃)₃F₃]⁻,
[P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻,
[P(C₃F₇)₃F₃]⁻, [P(C₂F₅)₂F₄]⁻, [(HO)₂P(O)O]⁻, [(CH₃O)₂P(O)O]⁻,
[(C₂H₅O)₂P(O)O]⁻, [(C₂F₅)₂P(O)O]⁻, [(C₂F₅)P(O)O₂]²⁻, [P(C₆F₅)₂F₄]⁻,
[(CH₃)₂P(O)O]⁻, [CH₃P(O)O₂]²⁻, [(CF₃)₂P(O)O]⁻, [CF₃P(O)O₂]²⁻,
[BF₃(CF₃)]⁻, [BF₂(C₂F₅)₂]⁻, [BF₃(C₂F₅)]⁻, [BF₂(CF₃)₂]⁻, [B(C₂F₅)₄]⁻,
[BF₃(CN)]⁻, [BF₂(CN)₂]⁻, [B(CN)₄]⁻, [B(OCH₃)₄]⁻, [B(CF₃)₄]⁻,
[B(OCH₃)₂(OC₂H₅)₂]⁻, [B(O₂C₂H₄)₂]⁻, [B(O₂C₂H₂)₂]⁻, [B(O₂C₆H₄)₂]⁻,
[N(CN)₂]⁻, [N(CF₃)₂]⁻, [SiF₆]⁻ or [SCN]⁻.

## Revendications

1. Procédé pour la préparation de sels de guanidinium de la formule (1) 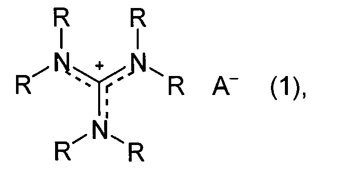 dans laquelle les substituants R, dans chaque cas indépendamment les uns des autres, présentent la signification de :
hydrogène,
alkyle en chaîne droite ou ramifié comportant 1-20 atomes de C,
cycloalkyle saturé, partiellement ou totalement non saturé comportant 3-7 atomes de C, lequel peut être substitué par des groupes alkyle comportant 1-6 atomes de C,
où un ou plusieurs substituants R peuvent être partiellement ou totalement substitués par halogène ou partiellement substitués par CN ou
NO₂ et halogène représente F, Cl, Br ou I,
où jusqu'à quatre substituants R peuvent être connectés les uns aux autres par paires au moyen d'une liaison simple ou double et où un atome de carbone ou deux atomes de carbone non adjacents d'un ou de plusieurs substituants R peut/peuvent être remplacé(s) par des atomes et/ou des groupes d'atomes choisis parmi le groupe -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O- et -P(R')₂=N-, où R' représente alkyle non fluoré, partiellement fluoré ou perfluoré comportant 1-6 atomes de C, cycloalkyle saturé ou partiellement non saturé comportant 3-7 atomes de C, phényle non substitué ou substitué ou un hétérocycle non substitué ou substitué
et
A⁻ est un sulfonate, alkyl- ou arylsulfate, hydrogènesulfate, imide, méthanide, carboxylate, phosphate, phosphinate, phosphonate, borate, thiocyanate, perchlorate, fluorosilicate ou nitrate,
par réaction d'un composé de la formule (2) 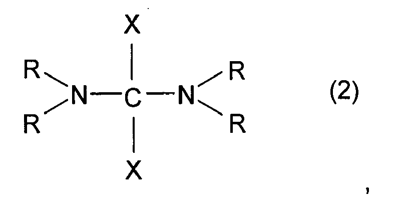
où les substituants R présentent une signification indiquée dans le cas de la formule (1) et X représente F, Cl ou Br,
avec un composé de la formule (3)
Kt⁺ A⁻ (3),
dans laquelle A⁻ présente une signification indiquée dans le cas de la formule (1) et
Kt⁺ peut être un proton, R"₃Si, un cation de métal alcalin ou alcalino-terreux, un cation d'ammonium, un cation de phosphonium ou un cation pris parmi le groupe 11 ou 12,
où R", dans chaque cas indépendamment des autres, représente phényle ou un groupe alkyle linéaire ou ramifié comportant 1-6 atomes de C, lequel peut être substitué par phényle,
et par réaction qui suit du composant résultant de la formule (4) 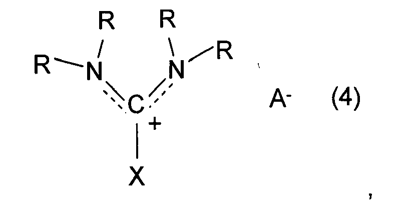
où les substituants R, X et A⁻ présentent une signification indiquée dans le cas de la formule (1) ou (2),
avec des composés de la formule (5) 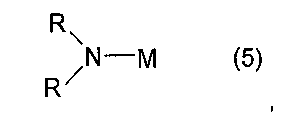
où les substituants R présentent une signification indiquée dans le cas de la formule (1) et
M représente hydrogène, un métal alcalin ou un métal alcalino-terreux.

2. Procédé selon la revendication 1, **caractérisé en ce que** des composés de la formule Kt⁺ A⁻ (3) sont utilisés, où Kt⁺ présente une signification indiquée dans le cas de la revendication 1 et
A⁻ est choisi parmi le groupe
[R¹OSO₃]⁻, [R¹SO₃]⁻, [R^{F}SO₃]⁻, [(FSO₂)₂N]⁻, [(R^{F}SO₂)2N]⁻, [(R^{F}SO₂)(R^{F}CO)N]⁻, [(R^{F}SO₂)₃C]⁻, [(FSO₂)₃C]⁻, [R¹CH₂C(O)O]⁻, [R^{F}C(O)O]⁻, [P(CₙF₂ₙ₊₁₋ₘHₘ)_{y}F_{6-y}]⁻, [P(C₆F₅)_{y}F_{6-y}]⁻, [(R¹O)₂P(O)O]⁻, [R¹₂P(O)O]⁻, [R¹P(O)O₂]²⁻, [R^{F}₂P(O)O]⁻, [R^{F}P(O)O₂]²⁻, [BF_{4-z}R^{F}_{z}]⁻, [BF_{4-z}(CN)_{z}]⁻, [B(C₆F₅)₄]⁻, [B(OR¹)₄]⁻, [N(CN)₂]⁻, [C(CN)₃]⁻, [N(CF₃)₂]⁻. [HSO₄]⁻, [SiF₆]²⁻, [ClO₄]⁻, [SCN]⁻ et [N0_{3]}⁻,
où les substituants R^{F}, dans chaque cas indépendamment des autres, présentent la signification de
alkyle perfluoré et en chaîne droite ou ramifié comportant 1-20 atomes de C,
alkényle perfluoré et en chaîne droite ou ramifié comportant 2-20 atomes de C et une ou plusieurs liaisons doubles,
cycloalkyle perfluoré et saturé, partiellement ou totalement non saturé comportant 3-7 atomes de C, lequel peut être substitué par des groupes perfluoroalkyle,
où les substituants R^{F} peuvent être connectés les uns aux autres par paires au moyen d'une liaison simple ou double et
où un atome de carbone ou deux atomes de carbone non adjacents du substituant R^{F} qui ne sont pas à la position α sur l'hétéroatome peut/peuvent être remplacé(s) par des atomes et/ou des groupes d'atomes choisis parmi le groupe -O-, -C(O)-, -S-, -S(O)-, -SO₂-, -N=, -N=N-, -NR'-, -PR'- et -P(O)R'-, où R' représente alkyle non fluoré, partiellement fluoré ou perfluoré comportant 1-6 atomes de C, cycloalkyle saturé ou partiellement non saturé comportant 3-7 atomes de C, phényle non substitué ou substitué ou un hétérocycle non substitué ou substitué,
où les substituants R¹, dans chaque cas indépendamment des autres, présentent la signification de
alkyle en chaîne droite ou ramifié comportant 1-20 atomes de C, alkényle en chaîne droite ou ramifié comportant 2-20 atomes de C et une ou plusieurs liaisons doubles,
alkynyle en chaîne droite ou ramifié comportant 2-20 atomes de C et une ou plusieurs liaisons triples,
cycloalkyle saturé, partiellement ou totalement non saturé comportant 3-7 atomes de C, lequel peut être substitué par des groupes alkyle comportant 1-6 atomes de C,
où les substituants R¹ peuvent être partiellement substitués par CN, NO₂ ou halogène et
halogène représente F, Cl, Br ou I,
où les substituants R¹ peuvent être connectés les uns aux autres par paires au moyen d'une liaison simple ou double et
où un atome de carbone ou deux atomes de carbone non adjacents des substituants R¹ qui ne sont pas à la position α sur l'hétéroatome peut/peuvent être remplacé(s) par des atomes et/ou des groupes d'atomes choisis parmi le groupe -O-, -C(O)-, -C(O)O-, -S-, -S(O)-, -SO₂-, -SO₃-, -N=, -N=N-, -NH-, -NR'-, -PR'-, -P(O)R'-, P(O)R'O-, OP(O)R'O-, -PR'₂=N-, -C(O)NH-, -C(O)NR'-, -SO₂NH- ou -SO₂NR'-, où R' représente alkyle non fluoré, partiellement fluoré ou perfluoré comportant 1-6 atomes de C, cycloalkyle saturé ou partiellement non saturé comportant 3-7 atomes de C, phényle non substitué ou substitué ou un hétérocycle non substitué ou substitué
et les variables
n représente 1 à 20,
m représente 0, 1, 2 ou 3,
y représente 0, 1, 2, 3 ou 4 et
z représente 0, 1, 2, 3 ou 4.

3. Procédé selon la revendication 1 ou 2, **caractérisé en ce que** A- est choisi parmi le groupe
[CH₃OSO₃]⁻, [C₂H₅OSO₃]⁻, [C(CN)₃]⁻,
[CH₃SO₃]⁻, [C₈H₁₇SO₃]⁻, [CH₃C₆H₄SO₃]⁻, [CF₃SO₃]⁻, [C₂H₅SO₃]⁻, [CF₃CF₂SO₃]⁻, [(CF₃SO₂)₂N]⁻, [(FSO₂)₂N]⁻, [(CF₃SO₂)(CF₃CO)N]⁻, [(C₂F₅SO₂)(CF₃CO)N]⁻, [(C₂F₅SO₂)₂N]⁻, ((CF₃SO₂)₃C]⁻, [(C₂F₅SO₂)₃C]⁻, [(FSO₂)₃C]⁻, [CH₃C(O)O]⁻, [C₂H₅C(O)O]⁻, [CF₃C(O)O]⁻, [CF₃CF₂C(O)O]⁻, [PF₆]⁻, [P(C₂F₅)₃F₃]⁻, [P(C₄F₉)₃F₃]⁻, [P(CF₃)₃F₃]⁻, [P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻, [P(C₃F₇)₃F₃]⁻, [P(C₂F₅)₂F₄]⁻, [(HO)₂P(O)O]⁻, [(CH₃O)₂P(O)O]⁻, [(C₂H₅O)₂P(O)O]⁻, [(C₂F₅)₂P(O)O]⁻, [(C₂F₅P(O)O₂]²⁻, [P(C₆F₅)₂F₄]⁻, [(CH₃)₂P(O)O]⁻, [CH₃P(O)O₂]²⁻, [(CF₃)₂P(O)O]⁻, [CF₃P(O)O₂]²⁻, [BF₄]⁻, [BF₃(CF₃)]⁻, [BF₂(C₂F₅)₂]⁻, [BF₃(C₂F₅)]⁻, [BF₂(CF₃)₂]⁻, [B(C₂F₅)₄]⁻, [BF₃(CN)]⁻, [BF₂(CN)₂]⁻, [B(CN)₄]⁻, [B(OCH₃)₄]⁻, [B(CF₃)₄]-, [B(OCH₃)₂(OC₂H₅)₂]⁻, [B(O₂C₂H₄)₂]⁻, [B(O₂C₂H₂)₂]⁻, [B(O₂C₆H₄)₂]⁻, [N(CN)₂] ⁻, [N(CF₃)₂]⁻, [HSO₄]⁻, [ClO₄]⁻, [SiF₆]⁻, [SCN]⁻ ou [NO₃]⁻.

4. Procédé selon une ou plusieurs des revendications 1 à 3, **caractérisé en ce que** le substituant X dans des composés dihalogène de la formule (2) selon la revendication 1 représente fluor ou chlore.

5. Procédé selon une ou plusieurs des revendications 1 à 4, **caractérisé en ce que** le substituant R dans des composés de la formule (5) selon la revendication 1, dans chaque cas indépendamment des autres, présente la signification de
hydrogène,
alkyle en chaîne droite ou ramifié comportant 1-20 atomes de C ou
cyloalkyle saturé, partiellement ou totalement non saturé comportant 3-7 atomes de C, lequel peut être substitué par des groupes alkyle comportant 1-6 atomes de C.

6. Procédé selon une ou plusieurs des revendications 1 à 5, **caractérisé en ce que** la première étape du procédé est mise en oeuvre dans l'eau.

7. Procédé selon une ou plusieurs des revendications 1 à 6, **caractérisé en ce que** la première étape du procédé est mise en oeuvre à des températures de 0° à 150°C.

8. Procédé selon une ou plusieurs des revendications 1 à 5, **caractérisé en ce que** la première étape du procédé est mise en oeuvre dans un solvant organique.

9. Procédé selon une ou plusieurs des revendications 1 à 5 et 8, **caractérisé en ce que** la première étape du procédé est mise en oeuvre à des températures de -50° à 150°C.

10. Procédé selon une ou plusieurs des revendications 1 à 9, **caractérisé en ce que** la seconde étape du procédé est mise en oeuvre sans solvant.

11. Procédé selon une ou plusieurs des revendications 1 à 10, **caractérisé en ce que** la seconde étape du procédé est mise en oeuvre à une température à laquelle au moins un composant est liquide.

12. Procédé selon une ou plusieurs des revendications 1 à 9, **caractérisé en ce que** la seconde étape du procédé est mise en oeuvre dans un solvant organique.

13. Procédé selon une ou plusieurs des revendications 1 à 9 et 12, **caractérisé en ce que** la seconde étape du procédé est mise en oeuvre à des températures de -50° à 150°C.

14. Procédé selon une ou plusieurs des revendications 1 à 9, **caractérisé en ce que** la seconde étape du procédé est mise en oeuvre dans l'eau.

15. Procédé selon une ou plusieurs des revendications 1 à 9 et 14, **caractérisé en ce que** la seconde étape du procédé est mise en oeuvre à des températures de 0° à 150°C.

16. Composés de la formule (4) 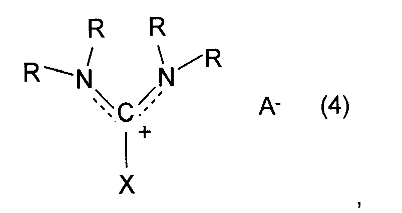 dans laquelle les substituants R, dans chaque cas indépendamment les uns des autres, présentent la signification de
hydrogène,
alkyle en chaîne droite ou ramifié comportant 1-20 atomes de C, cycloalkyle saturé, partiellement ou totalement non saturé comportant 3-7 atomes de C, lequel peut être substitué par des groupes alkyle comportant 1-6 atomes de C,
où un ou plusieurs substituants R peuvent être partiellement ou totalement substitués par halogène ou partiellement substitués par CN ou NO₂ et
halogène représente F, CI, Br ou I,
où jusqu'à quatre substituants R peuvent être connectés les uns aux autres par paires au moyen d'une liaison simple ou double et où un atome de carbone ou deux atomes de carbone non adjacents d'un ou de plusieurs substituants R peut/peuvent être remplacé(s) par des atomes et/ou des groupes d'atomes choisis parmi le groupe -O-, -C(O)-, -C(O)O-, -S-, -S(O)-, -SO₂-, -SO₃-, -N=, -N=N-, -NH-, -NR'-, -PR'-, -P(O)R'-, -P(O)R'-O-, -O-P(O)R'-O-, et -P(R')₂=N-, où R' représente alkyle non fluoré, partiellement fluoré ou perfluoré comportant 1-6 atomes de C, cycloalkyle saturé ou partiellement non saturé comportant 3-7 atomes de C, phényle non substitué ou substitué ou un hétérocycle non substitué ou substitué,
X représente F, Cl ou Br,
étant entendu que les quatre substituants R ne sont pas tous simultanément hydrogène et
A⁻ est choisi parmi le groupe
[R¹OSO₃]⁻, [R¹SO₃]⁻ , [R^{F}SO₃]⁻, [(FSO₂)₂N]⁻, [(R^{F}SO₂)(R^{F}CO)N]⁻, [(R^{F}SO₂)₃C]⁻, [(FSO₂)₃C]⁻, [R¹CH₂C(O)O]⁻, [P(CₙF₂ₙ₊₁₋ₘHₘ)_{y}F_{6-y}]⁻, [P(C₆F₅)_{y}F_{6-y}]⁻, [(R¹O)₂P(O)O]⁻, [R¹₂P(O)O]⁻, [R¹P(O)O₂]²⁻, [R^{F}₂P(O)O]⁻, [R^{F}P(O)O₂]²⁻, [BF_{4-z}R^{F}_{z}]⁻, [BF_{4-z}(CN)_{z}]⁻, [B(C₆F₅)₄]⁻, [B(OR¹)₄]⁻, [N(CN)₂]⁻, [(CN₃)C]⁻, [N(CF₃)₂]⁻, [SiF₆]²⁻ et [SCN]⁻,
où [CF₃SO₃]⁻ est exclu et
où les substituants R^{F}, dans chaque cas indépendamment des autres, présentent la signification de
alkyle perfluoré et en chaîne droite ou ramifié comportant 1-20 atomes de C,
alkényle perfluoré et en chaîne droite ou ramifié comportant 2-20 atomes de C et une ou plusieurs liaisons doubles,
cycloalkyle perfluoré et saturé, partiellement ou totalement non saturé comportant 3-7 atomes de C, lequel peut être substitué par des groupes perfluoroalkyle,
où les substituants R^{F} peuvent être connectés les uns aux autres par paires au moyen d'une liaison simple ou double et
où un atome de carbone ou deux atomes de carbone non adjacents du substituant R^{F} qui ne sont pas à la position α sur l'hétéroatome peut/peuvent être remplacé(s) par des atomes et/ou des groupes d'atomes choisis parmi le groupe -O-, -C(O)-, -S-, -S(O)-, -SO₂-, -N=, -N=N-, -NR'-, -PR'- et -P(O)R'-, où R' représente alkyle non fluoré, partiellement fluoré ou perfluoré comportant 1-6 atomes de C, cycloalkyle saturé ou partiellement non saturé comportant 3-7 atomes de C, phényle non substitué ou substitué ou un hétérocycle non substitué ou substitué,
où les substituants R¹, dans chaque cas indépendamment des autres, présentent la signification de
alkyle en chaîne droite ou ramifié comportant 1-20 atomes de C, alkényle en chaîne droite ou ramifié comportant 2-20 atomes de C et une ou plusieurs liaisons doubles,
alkynyle en chaîne droite ou ramifié comportant 2-20 atomes de C et une ou plusieurs liaisons triples,
cycloalkyle saturé, partiellement ou totalement non saturé comportant 3-7 atomes de C, lequel peut être substitué par des groupes alkyle comportant 1-6 atomes de C,
où les substituants R¹ peuvent être partiellement substitués par CN, NO₂ ou halogène et
halogène représente F, CI, Br ou I,
où les substituants R¹ peuvent être connectés les uns aux autres par paires au moyen d'une liaison simple ou double et
où un atome de carbone ou deux atomes de carbone non adjacents du substituant R¹ qui ne sont pas à la position α sur l'hétéroatome peut/ peuvent être remplacé() par des atomes et/ou des groupes d'atomes choisis parmi le groupe -O-, -C(O)-, -C(O)O-, -S-, -S(O)-, -SO₂-, -SO₃-, -N=, -N=N-, -NH-, -NR'-, -PR'-, -P(O)R'-, P(O)R'O-, OP(O)R'O-, -PR'₂=N-, -C(O)NH-, -C(O)NR'-, -SO₂NH- ou -SO₂NR'-, où R' représente alkyle non fluoré, partiellement fluoré ou perfluoré comportant 1-6 atomes de C, cycloalkyle saturé ou partiellement non saturé comportant 3-7 atomes de C, phényle non substitué ou substitué ou un hétérocycle non substitué ou substitué
et les variables
n représente 1 à 20,
m représente 0, 1, 2 ou 3,
y représente 1, 2, 3 ou 4 et
z représente 1, 2, 3 ou 4.

17. Composés selon la revendication 16, **caractérisés en ce que** les substituants R représentent hydrogène ou un groupe alkyle en chaîne droite ou ramifié comportant 1-12 atomes de C,
étant entendu que les quatre substituants R ne sont pas tous hydrogène ou qu'au moins deux substituants R sont connectés l'un à l'autre par des liaisons simples ou doubles pour former un cation monocyclique et
le contre-anion A⁻ représente
[CH₃OSO₃]⁻, [C₂H₅OSO₃]⁻, [C(CN)₃]⁻,
[CH₃SO₃]⁻, [C₈H₁₇SO₃]⁻, [CH₃C₆H₄SO₃]⁻, [C₂H₅SO₃]⁻, [CF₃CF₂SO₃]⁻,
[(FSO₂)₂N]⁻, [(CF₃SO₂)(CF₃CO)N]⁻, [(C₂F₅SO₂)(CF₃CO)N]⁻,
[(CF₃SO₂)₃C]⁻, [(C₂F₅SO₂)₃C]⁻, [(FSO₂)₃C]⁻, [CH₃C(O)O]⁻,
[C₂H₅C(O)O]⁻, [P(C₂F₅)₃F₃]⁻, [P(C₄F₉)₃F₃]⁻, [P(CF₃)₃F₃]⁻,
[P(C₂F₄H)(CF₃)₂F₃]⁻, [P(C₂F₃H₂)₃F₃]⁻, [P(C₂F₅)(CF₃)₂F₃]⁻, [P(C₆F₅)₃F₃]⁻,
[P(C₃F₇)₃F₃]⁻, [P(C₂F₅)₂F₄]⁻, [(HO)₂P(O)O]⁻, [(CH₃O)₂P(O)O]⁻,
[(C₂H₅O)₂P(O)O]⁻, [(C₂F₅)₂P(O)O]⁻, [(C₂F₅)P(O)O₂]²⁻, [P(C₆F₅)₂F₄]⁻,
[(CH₃)₂P(O)O]⁻, [CH₃P(O)O₂]²⁻, [(CF₃)₂P(O)O]⁻, [CF₃P(O)O₂]²⁻,
[BF₃(CF₃)]⁻, [BF₂(C₂F₅)₂]⁻, [BF₃(C₂F₅)] ⁻, [BF₂(CF₃)₂⁻, [B(C₂F₅)₄]⁻,
[BF₃(CN)]⁻, [BF₂(CN)₂]⁻, [B(CN)₄]⁻, [B(OCH₃)₄]⁻, [B(CF₃)₄]⁻,
[B(OCH₃)₂(OC₂H₅)₂]⁻, [B(O₂C₂H₄)₂]⁻, [B(O₂C₂H₂)₂]⁻, [B(O₂C₆H₄)₂]⁻,
[N(CN)₂] ⁻, [N(CF₃)₂]⁻, [SiF₆]⁻ ou [SCN]⁻.
